# EUROPEAN PATENT SPECIFICATION

(11) **EP 2 344 136 B1**
(45) Date of publication and mention of the grant of the patent: **15.06.2016**
(21) Application number: 09786269.2
(22) Date of filing: 17.09.2009
(51) Int. Cl.: A61K 9/16, A61K 31/485

(54) **PHARMACEUTICAL DOSAGE FORMS COMPRISING POLY(E-CAPROLACTONE)**
PHARMAZEUTISCHE DARREICHUNGSFORMEN MIT POLY(E-CAPROLACTON)
FORMES GALENIQUES COMPRENANT DE LA POLY(E-CAPROLACTONE)

(30) Priority: 18.09.2008 US 98089 P; 07.07.2009 US 223497 P
(43) Date of publication of application: 20.07.2011
(73) Proprietor: Purdue Pharma LP, Stamford, CT 06901-3431 (US)
(72) Inventor: MACHONIS, Meridith, Lee, Bridgewater NJ 08807-1259 (US)
(74) Representative: Ehlich, Eva Susanne
(86) International application number: PCT/IB2009/006917
(87) International publication number: WO 2010/032128

(56) References cited:
- US-A- 4 148 871
- US-A1- 2005 096 388
- BODMEIER R ET AL: "Preparation and characterization of microspheres containing the anti-inflammatory agents, indomethacin, ibuprofen, and ketoprofen" JOURNAL OF CONTROLLED RELEASE, ELSEVIER, AMSTERDAM, NL, vol. 10, no. 2, 1 November 1989 (1989-11-01), pages 167-175, XP025484215 ISSN: 0168-3659 [retrieved on 1989-11-01]

## Description

### TECHNICAL FIELD OF THE INVENTION

The present invention relates to pharmaceutical dosage forms, for example pharmaceutical dosage forms comprising poly(ε-caprolactone), and processes of manufacture, uses, and methods of treatment thereof.

### BACKGROUND OF THE INVENTION

Extended release oral dosage forms allow a specific release of active agent over an extended period of time. Larger dosing intervals, e.g. twice- or once-a-day dosing, may provide fewer side effects and overall better patient compliance.

Pharmaceutical products and in particular extended release dosage forms which usually comprise a larger amount of active agent in a single dose are increasingly the subject of abuse. For example, a particular dose of opioid agonist may be more potent when administered parenterally as compared to the same dose administered orally. Some formulations can be tampered with to provide the opioid agonist contained therein for illicit use. Controlled release opioid agonist formulations are sometimes milled or ground, and/or subject to extraction with solvents (e.g., ethanol) by drug abusers to provide the opioid contained therein for immediate release upon oral or parenteral administration.

Extended release opioid agonist dosage forms which can liberate a portion of the opioid upon exposure to ethanol, can also result in a patient receiving the dose more rapidly than intended if a patient concomitantly uses alcohol with the dosage form.

There continues to exist a need in the art for extended release pharmaceutical oral dosage forms. In particular there continues to exist a need for such dosage forms that resist illicit use and are safe when concomitantly used with alcohol.

Pitt et al. disclose in US 4,148,871 sustained subdermal delivery of drugs using poly(ε-caprolactone) and its copolymers, whereas Hunter et al. disclose in US 2005/0096388 compositions and methods for treating or preventing diseases of body passageways.

Further, the preparation and characterization of microspheres containing the anti-inflammatory agents, indomethacin, ibuprofen, and ketoprofen are disclosed by Bodmeier et al. in J. Controlled Release, 10(2), 1989, 167-175.

### OBJECTS AND SUMMARY OF THE INVENTION

It is an object of certain embodiments of the present invention to provide a solid oral extended release pharmaceutical dosage form, comprising a melt formed multi particulate extended release matrix formulation, comprising at least one poly(ε-caprolactone), and at least one active agent, wherein the active agent is an opioid analgesic.

It is a further object of certain embodiments of the present invention to provide a solid tamper resistant oral extended release pharmaceutical dosage form including an opioid analgesic which is resistant to milling and grinding.

It is a further object of certain embodiments of the present invention to provide a solid oral extended release pharmaceutical dosage form including an opioid analgesic which is resistant to milling, resistant to grinding and resistant to alcohol extraction.

According to certain embodiments the invention encompasses a solid extended release pharmaceutical dosage form, comprising a melt formed multi particulate extended release matrix formulation, comprising at least one poly(ε-caprolactone), and at least one active agent, wherein the active agent is an opioid analgesic, wherein at least one poly(ε-caprolactone) has an approximate number average molecular weight of at least 10,000.

According to certain embodiments the invention encompasses a solid extended release pharmaceutical dosage form, comprising a melt formed multi particulate extended release matrix formulation, comprising at least one poly(ε-caprolactone), and at least one active agent, wherein the active agent is an opioid analgesic, wherein poly(ε-caprolactone) is present at an amount of at least about 50 weight-% of the extended release matrix formulation.

According to certain embodiments the invention encompasses a solid extended release pharmaceutical dosage form, comprising a melt formed multi particulate extended release matrix formulation, comprising at least one poly(ε-caprolactone), and at least one active agent, wherein the active agent is an opioid analgesic, wherein the multi particulates have a diameter in the range of about 0.1 to about 3 mm.

According to certain embodiments the invention encompasses a solid extended release pharmaceutical dosage form, comprising a melt formed multi particulate extended release matrix formulation, comprising at least one poly(ε-caprolactone), and at least one active agent, wherein the active agent is an opioid analgesic, and additionally comprising at least one high molecular weight polyethylene oxide.

According to certain embodiments the invention encompasses a solid extended release pharmaceutical dosage form as described in the above paragraphs, wherein the active agent is an opioid analgesic, in particular selected from the group of codeine, morphine, oxycodone, hydrocodone, hydromorphone, or oxymorphone or pharmaceutically acceptable salts, hydrates and solvates thereof, and mixtures of any of the foregoing.

According to certain embodiments the invention encompasses a solid extended release pharmaceutical dosage form, comprising a melt formed multi particulate extended release matrix formulation, comprising at least one poly(ε-caprolactone), and at least one active agent, wherein the active agent is an opioid analgesic, wherein the dosage form provides release rates of the active agent in-vitro when measured by the USP Basket Method at 100 rpm at 900 ml simulated gastric fluid at 37 °C, between 12.5 % and 55 % (by wt) active agent released after 1 hour, between 25 % and 65 % (by wt) active agent released after 2 hours, between 45 % and 85 % (by wt) active agent released after 4 hours and between 55 % and 95 % (by wt) active agent released after 6 hours. These dosage forms comprise in particular oxycodone hydrochloride, hydromorphone hydrochloride, morphine sulfate or oxymorphone hydrochloride in the active agent.

According to certain other embodiments the invention encompasses a solid extended release pharmaceutical dosage form, comprising a melt formed multi particulate extended release matrix formulation, comprising at least one poly(ε-caprolactone), and at least one active agent, wherein the active agent is an opioid analgesic, wherein the dosage form provides release rates of the active agent in-vitro when measured by the USP Basket Method at 100 rpm at 900 ml simulated gastric fluid at 37 °C between 10 % and 30% (by wt) active agent released after 2 hour, 40 % and 75 % (by wt) active agent released after 8 hours and no less than 80%(by wt) active agent released after 22 hours.

Exemplary, a method of treatment may consist in administering a dosage form comprising an opioid analgesic as described herein for treatment of pain to a patient in need thereof.

The invention further encompasses the use of a dosage form comprising an opioid analgesic as described herein for use in the treatment of pain.

The invention further encompasses the use of poly(ε-caprolactone) as matrix forming material in the manufacture of a solid extended release dosage form comprising an active agent selected from opioids for imparting to the solid extended release dosage form resistance to milling.

The invention further encompasses a process of preparing a solid extended release pharmaceutical dosage form.

The invention further encompasses a solid extended release pharmaceutical dosage form obtainable by a process as described herein.

The term "solid extended release pharmaceutical dosage form" is preferably defined for purposes of the present invention as to refer to an oral dosage form.

The term "extended release" is defined for purposes of the present invention as to refer to products which are formulated to make the drug available over an extended period after ingestion thereby allowing a reduction in dosing frequency compared to a drug presented as a conventional dosage form (e.g. as a solution or an immediate release dosage form).

The term "immediate release" is defined for the purposes of the present invention as to refer to products which are formulated to allow the drug to dissolve in the gastrointestinal contents without substantial delay or prolongation of the dissolution or absorption of the drug.

The term "solid oral extended release pharmaceutical dosage form" for the purpose of the present invention refers to the administration form comprising a unit dose of active agent in extended release form such as an "extended release matrix formulation" and optionally other adjuvants and additives conventional in the art, such as a protective coating or a capsule and the like, and optionally any other additional features or components that are used in the dosage form. Unless specifically indicated the term "solid oral extended release pharmaceutical dosage form" refers to said dosage form in intact form i.e. prior to any tampering. The extended release pharmaceutical dosage form can e.g. be a tablet comprising the extended release matrix formulation or a capsule comprising the extended release matrix formulation in the form of multi particulates. The "extended release pharmaceutical dosage form" may comprise a portion of active agent in extended release form and another portion of active agent in immediate release form, e.g. as an immediate release layer of active agent surrounding the dosage form or an immediate release component included within the dosage form.

The term "extended release matrix formulation" is defined for purposes of the present invention as shaped solid form of a composition comprising at least one active agent and at least one extended release feature such as an extended release matrix material such as e.g. poly(ε-caprolactone). The composition can optionally comprise more than these two compounds namely further active agents and additional retardants and/or other materials, including but not limited to high molecular weight polyethylene oxides and other adjuvants and additives conventional in the art.

The term "poly(ε-caprolactone) "may for the purpose of the invention be abbreviated by PCL. The molecular weight of "poly(ε-caprolactone)" for the purpose of the present invention relates to a number average molecular weight. Poly(ε-caprolactone) is considered to have an approximate number average molecular weight of 10,000 when the viscosity is 400-1000 MPA at 25 degrees Celsius. Poly(ε-caprolactone) is considered to have an approximate number average molecular weight of 37,000 when the melt flow index is 40g/10 minutes at 160 degrees Celsius and 2.16kg. Poly(ε-caprolactone) is considered to have an approximate number average molecular weight of 42,500 when the melt flow index is 1.8 G/10 minutes at 80°C and 44 psi. Poly(ε-caprolactone) is considered to have an approximate number average molecular weight of 80,000 when the melt flow index is 1.0 G/10 minutes at 80 degrees Celsius and 44 psi..

The term "polyethylene oxide" may for the purpose of the invention be abbreviated by PEO. Preferably it has a molecular weight of at least 25,000, measured as is conventional in the art, and more preferably having a molecular weight of at least 100,000. Compositions with lower molecular weight are usually referred to as polyethylene glycols. WO2008/023261, describes pharmaceutical dosage forms prepared with PEO.

The term "high molecular weight polyethylene oxide" is defined for proposes of the present invention as having an approximate molecular weight of at least 1,000,000. For the purpose of this invention the approximate molecular weight is based on rheological measurements. Polyethylene oxide is considered to have an approximate molecular weight of 1,000,000 when a 2% (by wt) aqueous solution of said polyethylene oxide using a Brookfield viscometer Model RVF, spindle No. 1, at 10 rpm, at 25°C shows a viscosity range of 400 to 800 mPa s (cP). Polyethylene oxide is considered to have an approximate molecular weight of 2,000,000 when a 2% (by wt) aqueous solution of said polyethylene oxide using a Brookfield viscometer Model RVF, spindle No. 3, at 10 rpm, at 25°C shows a viscosity range of 2000 to 4000 mPa s (cP). Polyethylene oxide is considered to have an approximate molecular weight of 4,000,000 when a 1% (by wt) aqueous solution of said polyethylene oxide using a Brookfield viscometer Model RVF, spindle No. 2, at 2 rpm, at 25°C shows a viscosity range of 1650 to 5500 mPa s (cP). Polyethylene oxide is considered to have an approximate molecular weight of 5,000,000 when a 1% (by wt) aqueous solution of said polyethylene oxide using a Brookfield viscometer Model RVF, spindle No. 2, at 2 rpm, at 25°C shows a viscosity range of 5500 to 7500 mPa s (cP). Polyethylene oxide is considered to have an approximate molecular weight of 7,000,000 when a 1% (by wt) aqueous solution of said polyethylene oxide using a Brookfield viscometer Model RVF, spindle No. 2, at 2 rpm, at 25°C shows a viscosity range of 7500 to 10,000 mPa s (cP). Polyethylene oxide is considered to have an approximate molecular weight of 8,000,000 when a 1% (by wt) aqueous solution of said polyethylene oxide using a Brookfield viscometer Model RVF, spindle No. 2, at 2 rpm, at 25°C shows a viscosity range of 10,000 to 15,000 mPa s (cP). Regarding the lower molecular weight polyethylene oxides; Polyethylene oxide is considered to have an approximate molecular weight of 100,000 when a 5% (by wt) aqueous solution of said polyethylene oxide using a Brookfield viscometer Model RVT, spindle No. 1, at 50 rpm, at 25°C shows a viscosity range of 30 to 50 mPa s (cP) and polyethylene oxide is considered to have an approximate molecular weight of 900,000 when a 5% (by wt) aqueous solution of said polyethylene oxide using a Brookfield viscometer Model RVF, spindle No. 2, at 2 rpm, at 25°C shows a viscosity range of 8800 to 17,600 mPa s (cP).

The term "low molecular weight polyethylene oxide" is defined for purposes of the present invention as having, based on the rheological measurements outlined above, an approximate molecular weight of less than 1,000,000.

The term "melt formed" is defined for the purpose of the invention to relate to a process wherein an at least partially molten mass is formed and shaped. It includes without being limited to formed by extrusion, formed by casting and formed by injection molding.

The term "extrusion" is defined for purposes of the present invention as referring to a process by which material is mixed and at least partially melted then forced through a die under controlled conditions.

The term "casting" is defined for purposes of the present invention as referring to a process by which molten material is poured into a mold of a desired shape or onto a surface.

The term "injection molding" is defined for purposed of the present invention as referring to a process by which molten material is injected under pressure into a mold.

The term "direct compression" is defined for purposes of the present invention as referring to a tableting process wherein the tablet or any other compressed dosage form is made by a process comprising the steps of dry blending the compounds and compressing the dry blend to form the dosage form, e.g. by using a diffusion blend and/or convection mixing process (e.g. Guidance for Industry, SUPAC-IR/MR: Immediate Release and Modified Release Solid Oral Dosage Forms, Manufacturing Equipment Addendum).

For the purpose of certain embodiments of the present invention dosage forms are regarded as "resistant to milling" when the respective dosage form provides after milling an in-vitro dissolution rate, when measured in a USP Apparatus 1 (basket) at 100 rpm in 900 ml simulated gastric fluid at 37° C, characterized by the percent amount of active released at 1 hour of dissolution that deviates no more than about 20 % points from the corresponding in-vitro dissolution rate measured in a USP Apparatus 1 (basket) at 100 rpm in 900 ml simulated gastric fluid at 37° C without milling.

For the purpose of certain embodiments of the present invention dosage forms are regarded as "resistant to grinding" when the respective dosage form provides after grinding an in-vitro dissolution rate, when measured in a USP Apparatus 1 (basket) at 100 rpm in 900 ml simulated gastric fluid at 37° C, characterized by the percent amount of active released at 1 hour of dissolution that deviates no more than about 20 % points from the corresponding in-vitro dissolution rate measured in a USP Apparatus 1 (basket) at 100 rpm in 900 ml simulated gastric fluid at 37° C without grinding.

For the purpose of certain embodiments of the present invention dosage forms are regarded as "resistant to alcohol extraction" when the respective dosage form provides an in-vitro dissolution rate, when measured in a USP Apparatus 1 (basket) at 100 rpm in 900 ml simulated gastric fluid comprising 40% ethanol at 37° C, characterized by the percent amount of active released at 1 hour of dissolution that deviates no more than about 20 % points from the corresponding in-vitro dissolution rate measured in a USP Apparatus 1 (basket) at 100 rpm in 900 ml simulated gastric fluid at 37° C without ethanol.

For the purpose of certain embodiments of the present invention dosage forms are regarded as "resistant to milling and alcohol extraction" when the respective dosage form after milling provides an in-vitro dissolution rate, when measured in a USP Apparatus 1 (basket) at 100 rpm in 900 ml simulated gastric fluid comprising 40% ethanol at 37° C, characterized by the percent amount of active released at 1 hour of dissolution that deviates no more than about 20 % points from the corresponding in-vitro dissolution rate measured in a USP Apparatus 1 (basket) at 100 rpm in 900 ml simulated gastric fluid at 37° C without ethanol and without milling.

For the purpose of certain embodiments of the present invention dosage forms are regarded as "resistant to grinding and alcohol extraction" when the respective dosage form after grinding provides an in-vitro dissolution rate, when measured in a USP Apparatus 1 (basket) at 100 rpm in 900 ml simulated gastric fluid comprising 40% ethanol at 37° C, characterized by the percent amount of active released at 1 hour of dissolution that deviates no more than about 20 % points from the corresponding in-vitro dissolution rate measured in a USP Apparatus 1 (basket) at 100 rpm in 900 ml simulated gastric fluid at 37° C without ethanol and without grinding.

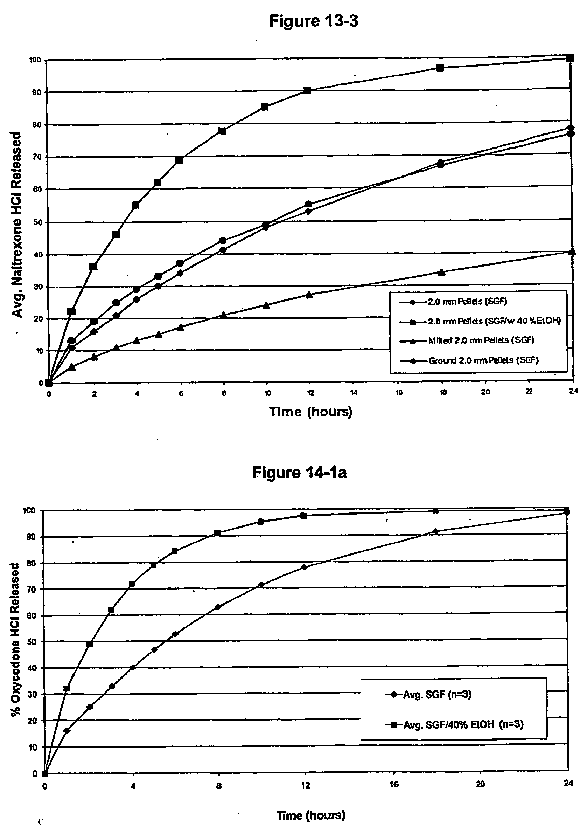

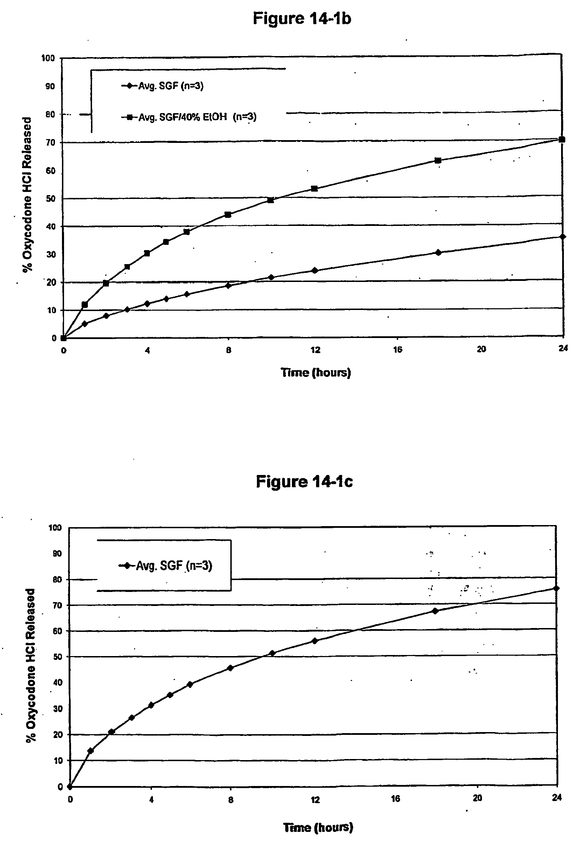

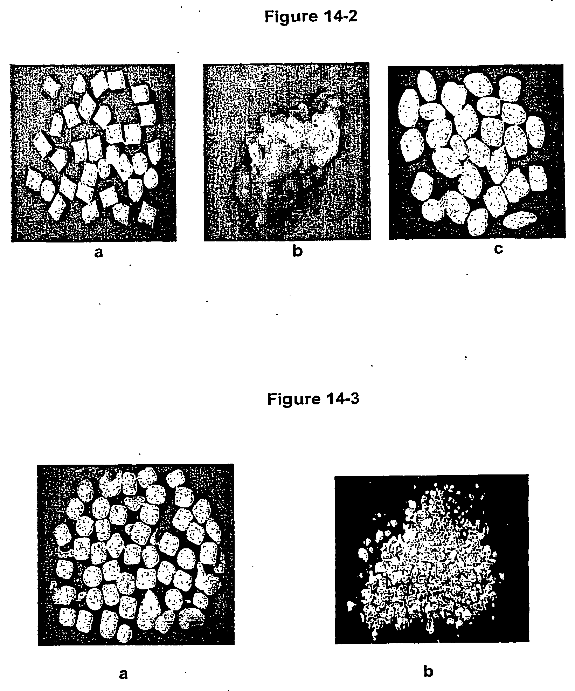

The term "milling" refers to the following procedure
Number of doses: 2
Duration of Milling: 15 seconds
Milling machine: IKA A11 Basic Impact Mill
Milling Chamber: Stainless steel
Chamber Volume: 80 ml
Blade: Stainless steel
Beater: Stainless steell.4034
Rotor Shaft: Stainless steel 1.4571
MotorSpeed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W
For demonstration purposes only and irrelevant for the definition of milling resistance, milling can also be performed in a coffee mill. For demonstration sake Figure 14-3 shows the multi particulates of the present invention and a comparison tablet after milling in a coffee mill.

The term "grinding" refers to the following procedure:
Equipment: 8 oz Glass Mortar with Pestle
Number of doses: 2
Duration of grinding: 20 rotations

The term "Simulated Gastric Fluid" (SGF) relates to Simulated Gastric Fluid without enzymes and either without sodium lauryl sulfate (SLS), with 0.5% sodium lauryl sulfate or 0.1% sodium lauryl sulfate. The term "Simulated Gastric Fluid with 40% Ethanol" relates to SGF with 40% Ethanol and without enzymes and without sodium lauryl sulfate.

For the purpose of the present invention the term "active agent" is defined as a pharmaceutically active substance which includes opioid analgesics.

For purposes of the present invention, the term "opioid analgesic" includes single compounds and combinations of compounds selected from the group of opioids and which provide an analgesic effect such as one single opioid agonist or a combination of opioid agonists, one single mixed opioid agonist-antagonist or a combination of mixed opioid agonist-antagonists, or one single partial opioid agonist or a combination of partial opioid agonists and combinations of an opioid agonists, mixed opioid agonist-antagonists and partial opioid agonists with one ore more opioid antagonists, stereoisomers, ether or ester, salts, hydrates and solvates thereof, compositions of any of the foregoing, and the like.

The present invention disclosed herein is specifically meant to encompass the use of the opioid analgesic in form of any pharmaceutically acceptable salt thereof.

Pharmaceutically acceptable salts include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate, and the like; amino acid salts such as arginate, asparginate, glutamate and the like, and metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like.

The opioids used according to the present invention may contain one or more asymmetric centers and may give rise to enantiomers, diastereomers, or other stereoisomeric forms. The present invention is also meant to encompass the use of all such possible forms as well as their racemic and resolved forms and compositions thereof. When the compounds described herein contain olefinic double bonds or other centers of geometric asymmetry, it is intended to include both E and Z geometric isomers. All tautomers are intended to be encompassed by the present invention as well.

As used herein, the term "stereoisomers" is a general term for all isomers of individual molecules that differ only in the orientation of their atoms is space. It includes enantiomers and isomers of compounds with more than one chiral center that are not mirror images of one another (diastereomers).

The term "chiral center" refers to a carbon atom to which four different groups are attached.

The term "enantiomer" or "enantiomeric" refers to a molecule that is nonsuperimposeable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule.

Opioid agonists useful in the present invention include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts, hydrates and solvates thereof, mixtures of any of the foregoing, and the like.

Opioid antagonists useful in combination with opioid agonists as described above are e.g. naloxone, naltrexone and nalmephene or pharmaceutically acceptable salts, hydrates and solvates thereof, mixtures of any of the foregoing, and the like.

In certain embodiments e.g. a combination of oxycodone HCl and naloxone HCl in a ratio of about 2:1 is used. Examples for ratios of oxycodone HCl : naloxone HCl are 5: 2.5, 10:5, 20:10, 30:15, 40:20, 60:30, 80:40, 100:50 and 120:60.

In certain embodiments, the opioid analgesic is selected from codeine, morphine, oxycodone, hydrocodone, hydromorphone, or oxymorphone or pharmaceutically acceptable salts, hydrates and solvates thereof, mixtures of any of the foregoing, and the like.

In certain embodiments, the opioid analgesic is oxycodone, hydromorphone or oxymorphone or a salt thereof such as e.g. the hydrochloride. The dosage form comprises from about 5 mg to about 500 mg oxycodone hydrochloride, from about 1 mg to about 100 mg hydromorphone hydrochloride or from about 5 mg to about 500 mg oxymorphone hydrochloride. If other salts, derivatives or forms are used, equimolar amounts of any other pharmaceutically acceptable salt or derivative or form including but not limited to hydrates and solvates or the free base may be used. The dosage form may comprise e.g. 5 mg, 7.5 mg, 10 mg, 15 mg, 20 mg, 30 mg, 40 mg, 45 mg, 50 mg, 60 mg, or 80 mg, 90 mg, 100 mg, 120 mg or 160 mg oxycodone hydrochloride or equimolar amounts of any other pharmaceutically acceptable salt, derivative or form including but not limited to hydrates and solvates or of the free base. The dosage form may comprise e.g. 5 mg, 7.5 mg, 10 mg, 15 mg, 20 mg, 30, mg, 40 mg, 45 mg, 50 mg, 60 mg, or 80 mg, 90 mg, 100 mg, 120 mg or 160 mg oxymorphone hydrochloride or equimolar amounts of any other pharmaceutically acceptable salt, derivative or form including but not limited to hydrates and solvates or of the free base. The dosage form may comprise e.g. 2 mg, 4 mg, 5 mg, 8 mg, 12 mg, 15 mg, 16 mg, 24 mg, 25 mg, 32 mg, 48 mg, 50 mg, 64 mg or 75 mg hydromorphone hydrochloride or equimolar amounts of any other pharmaceutically acceptable salt, derivative or form including but not limited to hydrates and solvates or of the free base.

WO 2005/097801 A1, US 7,129,248 B2 and US 2006/0173029 A1, describe a process for preparing oxycodone hydrochloride having a 14-hydroxycodeinone level of less than about 25 ppm, preferably of less than about 15 ppm, less than about 10 ppm, or less than about 5 ppm, more preferably of less than about 2 ppm, less than about 1 ppm, less than about 0.5 ppm or less than about 0.25 ppm.

The term "ppm" as used herein means "parts per million". Regarding 14-hydroxycodeinone, "ppm" means parts per million of 14-hydroxycodeinone in a particular sample product. The 14-hydroxycodeinone level can be determined by any method known in the art, preferably by HPLC analysis using UV detection.

In certain embodiments of the present invention, wherein the active agent is oxycodone hydrochloride, oxycodone hydrochloride is used having a 14-hydroxycodeinone level of less than about 25 ppm, preferably of less than about 15 ppm, less than about 10 ppm, or less than about 5 ppm, more preferably of less than about 2 ppm, less than about 1 ppm, less than about 0.5 ppm or less than about 0.25 ppm.

In certain other embodiments other therapeutically active agents may be used in accordance with the present invention, in combination with opioids. Examples of such therapeutically active agents include antihistamines (e.g., dimenhydrinate, diphenhydramine, chlorpheniramine and dexchlorpheniramine maleate), non -steroidal anti-inflammatory agents (e.g., naproxen, diclofenac, indomethacin, ibuprofen, sulindac, Cox-2 inhibitors) and acetaminophen, anti-emetics (e.g., metoclopramide, methylnaltrexone), antiepileptics (e.g., phenytoin, meprobmate and nitrazepam), vasodilators (e.g., nifedipine, papaverine, diltiazem and nicardipine), anti-tussive agents and expectorants (e.g. codeine phosphate), anti-asthmatics (e.g. theophylline), antacids, anti-spasmodics (e.g. atropine, scopolamine), antidiabetics (e.g., insulin), diuretics (e.g., ethacrynic acid, bendrofluthiazide), anti-hypotensives (e.g., propranolol, clonidine), antihypertensives (e.g., clonidine, methyldopa), bronchodilatiors (e.g., albuterol), steroids (e.g., hydrocortisone, triamcinolone, prednisone), antibiotics (e.g., tetracycline), antihemorrhoidals, hypnotics, psychotropics, antidiarrheals, mucolytics, sedatives, decongestants (e.g. pseudoephedrine), laxatives, vitamins, stimulants (including appetite suppressants such as phenylpropanolamine) and cannabinoids, as well as pharmaceutically acceptable salts, hydrates, and solvates of the same.

In certain embodiments, the invention is directed to Cox-2 inhibitors as active agents, in combination with opioid analgesics, for example the use Cox-2 inhibitors such as meloxicam (4-hydroxy-2-methyl-*N*-(5-methyl-2-thiazolyl)-2*H*-1,2-benzothiazine-3-carboxamide-1,1-dioxide), as disclosed in U.S. Serial No. 10/056,347 and 11/825,938, nabumetone (4-(6-methoxy-2-naphthyl)-2-butanone), as disclosed in U.S. Serial No. 10/056,348, celecoxib (4-[5-(4-methylphenyl)-3-(trifluoromethyl)-1H-pyrazol-1-yl]benzenesulfonamide), as disclosed in U.S. Serial No. 11/698,394, nimesulide (N-(4-Nitro-2-phenoxyphenyl)methanesulfonamide), as disclosed in U.S. Serial No. 10/057,630, and N-[3-(formylamino)-4-oxo-6-phenoxy-4H-1-benzopyran-7-yl] methanesulfonamide (T-614), as disclosed in U.S. Serial No. 10/057,632.

The present invention is also directed to the dosage forms utilizing active agents such as for example, benzodiazepines, barbiturates or stimulants such as amphetamines. These may be combined with the respective antagonists.

The term "benzodiazepines" refers to benzodiazepines and drugs that are derivatives of benzodiazepine that are able to depress the central nervous system. Benzodiazepines include, but are not limited to, alprazolam, bromazepam, chlordiazepoxide, clorazepate, diazepam, estazolam, flurazepam, halazepam, ketazolam, lorazepam, nitrazepam, oxazepam, prazepam, quazepam, temazepam, triazolam, methylphenidate as well as pharmaceutically acceptable salts, hydrates, and solvates and mixtures thereof. Benzodiazepine antagonists that can be used in the present invention include, but are not limited to, flumazenil as well as pharmaceutically acceptable salts, hydrates, and solvates.

Barbiturates refer to sedative-hypnotic drugs derived from barbituric acid (2,4,6-trioxohexahydropyrimidine). Barbiturates include, but are not limited to, amobarbital, aprobarbotal, butabarbital, butalbital, methohexital, mephobarbital, metharbital, pentobarbital, phenobarbital, secobarbital and as well as pharmaceutically acceptable salts, hydrates, and solvates mixtures thereof. Barbiturate antagonists that can be used in the present invention include, but are not limited to, amphetamines as well as pharmaceutically acceptable salts, hydrates, and solvates.

Stimulants refer to drugs that stimulate the central nervous system. Stimulants include, but are not limited to, stimulants such as amphetamines, such as amphetamine, dextroamphetamine resin complex, dextroamphetamine, methamphetamine, methylphenidate as well as pharmaceutically acceptable salts, hydrates, and solvates and mixtures thereof. Stimulant antagonists that can be used in the present invention include, but are not limited to, benzodiazepines, as well as pharmaceutically acceptable salts, hydrates, and solvates as described herein.

### BRIEF DESCRIPTION OF THE DRAWINGS

Fig. 1 to 14-1 depict the dissolution profiles of the respective Examples 1 to 14 as described below.
Fig .14-2 depicts the intact (a), milled (b) and grinded(c) multiparticulates of Example 14
Fig. 14-3 depicts the multiparticulates of Example 14 after milling in a coffee mill (a) and a comparison tablet after milling in a coffee mill (b).

### DETAILED DESCRIPTION

According to certain embodiments the invention relates to a solid extended release pharmaceutical dosage form, comprising a melt formed multi particulate extended release matrix formulation, comprising at least one poly(ε-caprolactone), and at least one active agent, wherein the active agent is an opioid analgesic.

The inventors have found that poly(ε-caprolactone) is a suitable polymeric material for forming an extended release matrix formulation which can provide a wide variety of release profiles when used in the form of melt formed multi particulates. The melt forming according to the invention can be accomplished by several methods, including extrusion, casting and injection molding. The multi particulates have preferably a diameter in the range of about 0.1 to about 3 mm.

Without wanting to be bound to any theory, it has also been found that poly(ε-caprolactone), due to its specific polymer characteristics, imparts a milling and/or grinding resistance to the extended release formulation in that the multi particles comprising poly(ε-caprolactone) do not form during milling and/or grinding smaller individual particles but in case of milling tend to fuse/melt together forming a lumpy mass and in case of grinding might deform. This is shown by Figures 14-2 and 14-3. It is believed that the release of the active agent does therefore not substantially change upon milling or grinding. In some cases the release is even slowed down. Thereby the extended release dosage form comprising said multi particulates is rendered less attractive for abuse.

According to certain embodiments of the invention at least one poly(ε-caprolactone) with an approximate number average molecular weight of at least about 6,000 is used. According to certain embodiments of the invention the at least one poly(ε-caprolactone) has an approximate number average molecular weight of at least about 10,000. According to certain embodiments of the invention the at least one poly(ε-caprolactone) has an approximate number average molecular weight of at least about 20,000. According to certain embodiments of the invention the at least one poly(ε-caprolactone) has an approximate number average molecular weight of at least about 25,000. According to certain embodiments of the invention the at least one poly(ε-caprolactone) has an approximate number average molecular weight of at least about 37,000. According to certain embodiments of the invention the at least one poly(ε-caprolactone) has an approximate number average molecular weight of about 42,500. According to certain embodiments of the invention the at least one poly(ε-capiolactone) has an approximate number average molecular weight of at least about 80,000. According to further certain embodiments of the invention, the at least one pbly(ε-caprolactone) has an approximate number average molecular weight of between about 6,000 to about 80,000, or between about 10,000 and about 80,000, or between about 20,000 and about 80,000, or between about 25,000 and about 80,000or between about 37,000 and about 80,000, or between about 42,500 and about 80,000.

According to certain embodiments of the invention the extended release matrix formulation comprises at least two poly(ε-caprolactone) with an approximate number average molecular weight of between about 6,000 and about 25,000 and between about 37,000 and about 80,000, or between about 10,000 and about 25,000 and between about 37,000 and about 80,000, or between about 10,000 and about 25,000 and between about 42,500 and about 80,000.

According to certain embodiments of the invention in the extended release matrix formulation the overall content of poly(ε-caprolactone) is at least about 50 weight-%, or at least about 60 weight-%, or at least about 70 weight-%, or at least about 80 weight-%, or at least about 90 weight-%, or between about 50 and about 90 weight-%, or between about 60 and about 90 weight-%, or between about 70 and about 90 weight-%, or between about 80 and about 90 weight-% of the extended release matrix formulation.

According to certain embodiments of the invention the extended release matrix formulation comprises least one poly(ε-caprolactone) with an approximate number average molecular weight of between about 37,000 and about 80,000 which is present at an amount of between about 50 and about 90 weight-% of the extended release matrix formulation.

According to certain embodiments of the invention the extended release matrix formulation comprises further at least one polyethylene glycol, which may be present at an amount of between about 1 and about 20 or about 1 and about 15 weight-%.

According to certain embodiments of the invention the extended release matrix formulation comprises further at least one high molecular weight polyethylene oxide with an approximate molecular weight of between about 1,000,000 and about 10,000,000, based on rheological measurements. It is the finding of the inventors that the combination of poly(ε-caprolactone) and high molecular polyethylene oxide provide a resistance to milling and/or grinding in combination with a resistance to alcohol extraction thereby rendering the dosage form less attractive for illicit use and rendering the dosage form safer when used in combination with alcohol. The high molecular weight polyethylene oxide may be present at an amount of between about 5 and about 35 weight-%.

According to certain such embodiments of the invention the high molecular weight polyethylene oxide used has been screened through a screen with a size of 15/100 of the average diameter of the resulting melt formed multi particulate extended release formulation. According to certain embodiments the high molecular weight polyethylene oxide used has been screened with a 100 US mesh screen.

According to certain embodiments of the invention the extended release matrix formulation further comprises at least one poloxamer. The extended release matrix formulations may comprise further any other ingredients/excipients as conventional in the art.

According to certain embodiments of the invention the active agent is an opioid analgesic, in particular selected from the group of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts, hydrates and solvates thereof, mixtures of any of the foregoing. According to certain preferred embodiments of the invention the opioid analgesic is selected from the group of codeine, morphine, oxycodone, hydrocodone, hydromorphone, or oxymorphone or pharmaceutically acceptable salts, hydrates and solvates thereof, mixtures of any of the foregoing.

According to certain embodiments of the invention the opioid analgesic is oxycodone hydrochloride and the dosage form comprises from about 5 mg to about 500 mg of oxycodone hydrochloride or in particular comprises 5 mg, 7.5 mg, 10 mg, 15 mg, 20 mg, 30, mg, 40 mg, 45 mg, 50 mg, 60 mg, or 80 mg, 90 mg, 100 mg, 120 mg or 160 mg of oxycodone hydrochloride. According to certain such embodiments the oxycodone hydrochloride has a 14-hydroxycodeinone level of less than about 25 ppm, preferably of less than about 15 ppm, less than about 10 ppm, or less than about 5 ppm, or even less than 1 ppm.

According to certain embodiments of the invention the opioid analgesic is oxymorphone hydrochloride and the dosage form comprises from about 1 mg to about 500 mg of oxymorphone hydrochloride, in particular 5 mg, 7.5 mg, 10 mg, 15 mg, 20 mg, 30 mg, 40 mg, 45 mg, 50mg, 60 mg, or 80 mg, 90 mg, 100 mg, 120 mg or 160 mg of oxymorphone hydrochloride.

According to certain embodiments of the invention the opioid analgesic is hydromorphone hydrochloride and the dosage form comprises from about 1 mg to about 100 mg of hydromorphone hydrochloride, in particular 2 mg, 4 mg, 5 mg, 8 mg, 12 mg, 15 mg, 16 mg, 24 mg, 25 mg, 32 mg, 48 mg 50 mg, 64 mg or 75 mg of hydromorphone hydrochloride.

According to certain embodiments of the invention the dosage form contains active in immediate release form, wherein the same or different active agents are in extended release and in immediate release form.

According to certain embodiments of the invention the dosage form provides release rates of the active agent in-vitro when measured by the USP Basket Method at 100 rpm at 900 ml simulated gastric fluid at 37°C, between about 12.5 % and about 55 % (by wt) active agent released after 1 hour, between about 25 % and about 65 % (by wt) active agent released after 2 hours, between about 45 % and about 85 % (by wt) active agent released after 4 hours and between about 55 % and about 95 % (by wt) active agent released after 6 hours.

According to certain embodiments of the invention the dosage form provides release rates of the active agent in-vitro when measured by the USP Basket Method at 100 rpm at 900 ml simulated gastric fluid at 37 °C between about 10 % and about 30% (by wt) active agent released after 2 hour, about 40 % and about 75 % (by wt) active agent released after 8 hours and no less than about 80%(by wt) active agent released after 22 hours.

According to certain embodiments of the invention the dosage form provides an in-vitro dissolution rate, when measured in a USP Apparatus 1 (basket) at 100 rpm in 900 ml simulated gastric fluid comprising 40% ethanol at 37° C, characterized by the percent amount of active agent released at 1 hour of dissolution that deviates no more than about 20 % points or no more than about 10 % points from the corresponding in-vitro dissolution rate measured in a USP Apparatus 1 (basket) at 100 rpm in 900 ml simulated gastric fluid at 37° C without ethanol.

According to certain embodiments of the invention the dosage form provides after milling an in-vitro dissolution rate, when measured in a USP Apparatus 1 (basket) at 100 rpm in 900 ml simulated gastric fluid at 37° C, characterized by the percent amount of active agent released at 1 hour of dissolution that increases no more than about 20 % points or no more than 10 % points or even decreases when compared to the corresponding in-vitro dissolution rate measured in a USP Apparatus 1 (basket) at 100 rpm in 900 ml simulated gastric fluid at 37° C without milling.

According to certain embodiments of the invention the dosage form provides after grinding an in-vitro dissolution rate, when measured in a USP Apparatus 1 (basket) at 100 rpm in 900 ml simulated gastric fluid at 37° C, characterized by the percent amount of active agent released at 1 hour of dissolution that increases no more than about 20 % points or no more than about 10 % points or even decreases when compared to the corresponding in-vitro dissolution rate measured in a USP Apparatus 1 (basket) at 100 rpm in 900 ml simulated gastric fluid at 37° C without grinding.

According to certain embodiments of the invention the dosage form after milling provides an in-vitro dissolution rate, when measured in a USP Apparatus 1 (basket) at 100 rpm in 900 ml simulated gastric fluid comprising 40% ethanol at 37° C, characterized by the percent amount of active agent released at 1 hour of dissolution that deviates no more than about 20 % points or no more than 10 % points from the corresponding in-vitro dissolution rate measured in a USP Apparatus 1 (basket) at 100 rpm in 900 ml simulated gastric fluid without ethanol at 37° C without milling.

According to certain embodiments of the invention the dosage form after grinding provides an in-vitro dissolution rate, when measured in a USP Apparatus 1 (basket) at 100 rpm in 900 ml simulated gastric fluid, comprising 40% ethanol, at 37° C, characterized by the percent amount of active agent released at 1 hour of dissolution that deviates no more than about 20 % points or no more than 10 % points from the corresponding in-vitro dissolution rate measured in a USP Apparatus 1 (basket) at 100 rpm in 900 ml simulated gastric fluid without ethanol at 37° C without grinding.

According to certain such embodiments of the invention as described in the paragraphs [0086] to [0092] above the active agent is oxycodone hydrochloride, hydromorphone hydrochloride or oxymorphone hydrochloride.

According to a certain aspect of the invention the dosage forms as described herein are for used in the treatment of pain in a patient in need thereof, wherein the dosage form comprises an opioid analgesic .

According to a certain aspect of the invention poly(ε-caprolactone) is used as matrix forming material in the manufacture of a solid extended release oral dosage form comprising an active agent selected from opioids for imparting to the solid extended release oral dosage form resistance to milling.

According to a certain aspect of the invention a process of preparing a solid oral extended release pharmaceutical dosage form is provided comprising the steps of:
- Melting and blending the poly(ε-caprolactone)_and possible further ingredients except the active agent on a Thermodyne Hot Plate (temperature range about 90° - about 160°C) for optionally approximately 3 minutes to obtain a mixture;
- Adding the active agent to the mixture on the Thermodyne Hot Plate (temperature range about 90° - about 160°C) until the mixture appeared homogeneous to obtain a blend;
- Casting the molten blend by pressing between two stainless steel plates to a thickness of optionally approximately 2 millimeters and cooling to room temperature to obtain a sheet; and
- Pelletizing the sheet by cutting into pellets of optionally approximately 2 mm in length and width.

According to a certain aspect of the invention a process of preparing a solid oral extended release pharmaceutical dosage form is provided comprising the steps of:
- Screening active agent, poly(ε-caprolactone) and optionally other ingredients through a # 20 US mesh screen;
- Blending the screened materials for optionally 10 minutes at ambient temperature;
- Extruding the screened and blended materials in a twin screw extruder fitted with a die and set on counter-rotation with zone (barrel) temperatures ranged from about 18°C to about 110°C to obtain strands with a
   Leistritz - ZSE 27 Twin Screw Extruder (Counter-Rotation)
   Neslab Model CFT-150 Chiller
   Accurate Powder Feeder
   Domer 8-foot Conveyor
   Grablab Electronic Timer
- Cooling the strands to ambient temperature;
- Pelletizing the cooled strands into pellets.
In such a process the polyethylene oxide may be screened through a # 100 US mesh screen.

According to a further aspect of the invention the solid oral extended release pharmaceutical dosage form is obtainable by a process as described above.

### EXAMPLES

The present invention will now be more fully described with reference to the accompanying examples.

### REFERENCE EXAMPLE 1

The composition of the poly(ε-caprolactone) multiparticulates is summarized in Table 1 below.

**Table 1**

| **.Ingredient (Trade Name)** | **Amt/unit (mg)** | **Amt/batch (g)** |
|---|---|---|
| Naltrexone HCl | 12.0 | 2.4 |
| Poly(ε-caprolactone), Mn ∼ 42500 | 61.0 | 12.2 |
| Butylated Hydroxytoluene | 1.0 | 0.2 |
| Total | 74.0 | 14.8 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 3 minutes.
3. Melting and Blending: The Naltrexone HCl was slowly added to the PCL/BHT and mixed on a Thermodyne Thermodyne Hot Plate (temperature range 90° - 160°C) for approximately 3 minutes.
4. Casting: The molten drug/polymer blend was pressed between two stainless steel plates to a thickness of approximately 2 millimeters.
5. Cooling: The drug/polymer blend was cooled at room temperature.
6. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid with 0.1% Sodium Lauryl Sulfate or 900 ml Simulated Gastric Fluid with 40% ethanol.
4. Analytical Method - UV Analysis, UV/Vis Spectrophotometer setup with flow through cells (wavelength 230 nm). Peristaltic pump (flow rate approx 5ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

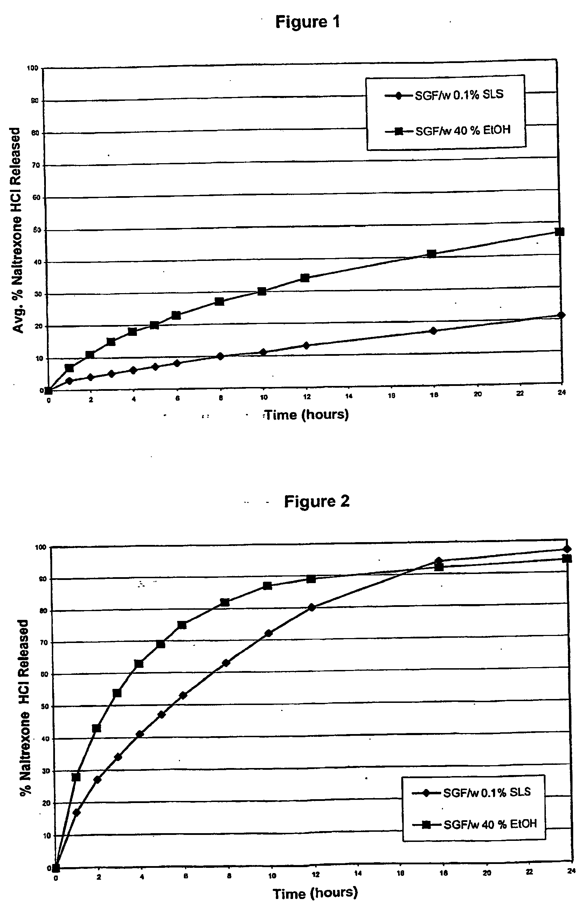

The dissolution results for the poly(ε-caprolactone) multiparticules are summarized in Figure 1 and Table 1a.

**Table 1a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1h | 2h | 3h | 4h | 5h | 6h | 8h | 10h | 12 h | 18h | 24 h |
| **SGF/w 0.1% SLS** | 3 | 4 | 5 | 6 | 7 | 8 | 10 | 11 | 13 | 17 | 21 |
| **SGF with 40% EtOH** | 7 | 11 | 15 | 18 | 20 | 23 | 27 | 30 | 34 | 41 | 47 |

### Milling Procedure

Equipment: IKA A11 basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind and fused/melted during milling.

### REFERENCE EXAMPLE 2

The composition of the poly(ε-caprolactone) multiparticulates is summarized in Table 2 below.

**Table 2**

| **Ingredient (Trade Name)** | **Amt/unit (mg)** | **Amt/batch (g)** |
|---|---|---|
| Naltrexone HCl | 12.0 | 1.2 |
| Poly(ε-caprolactone), Mn ∼ 42500 | 48.4 | 4.84 |
| Polyethylene Glycol 3350 (Carbowax PEG 3350) | 10.1 | 1.01 |
| Butylated Hydroxytoluene | 1.8 | 0.18 |
| Total | 72.3 | 7.23 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 3 minutes.
3. Melting and Blending: The polyethylene glycol (PEG 3350) was melted and mixed with the PCL/BHT mixture on a Thermodyne Thermodyne Hot Plate (temperature range 90° - 160°C) for approximately 3 minutes.
4. Weighing: The resulting polymer blend was weighed to determine the amount of PEG incorporated with Mettler, Sartorious balances.
5. Melting and Blending: The polymer blend was melted (temperature range 90°-160°C). The Naltrexone HCl was slowly added to the molten polymer blend and mixed on a Thermodyne Thermodyne Hot Plate (temperature range 90° - 160°C) for approximately 3 minutes.
6. Casting: The molten drug/polymer blend was pressed between two stainless steel plates to a thickness of approximately 2 millimeters.
7. Cooling: The drug/polymer blend was cooled at room temperature.
8. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid with 0.1% Sodium Lauryl Sulfate or 900 ml Simulated Gastric Fluid with 40% ethanol.
4. Analytical Method - UV Analysis, UV/Vis Spectrophotometer setup with flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

The dissolution results for the poly(ε-caprolactone) multiparticules are summarized in Figure 2 and Table 2a.

**Table 2a**

| | **Dissolution Result** | | | | | | | | | | |
|---|---|---|---|---|---|---|---|---|---|---|---|
| **Dissolution Media** | **Naltrezone HCl % Released (n=1)** | | | | | | | | | | |
| | 1 h | 2 h | 3 h | 4 h | 5 h | 6 h | 8 h | 10 h | 12 h | 18 h | 24 h |
| **SGF/w 0.1% SLS** | 17 | 26 | 34 | 41 | 47 | 53 | 63 | 72 | 80 | 94 | 97 |
| **SGF with 40% EtOH** | 28 | 43 | 54 | 63 | 69 | 75 | 82 | 87 | 89 | 92 | 94 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind but did not fuse/melt during milling.

### REFERENCE EXAMPLE 3

The composition of the poly(ε-caprolactone) multi particulates is summarized in Table 3 below.

**Table 3**

| **Ingredient (Trade Name)** | **Amt/unit (mg)** | **Amt/batch (g)** |
|---|---|---|
| Naltrexone HCl | 12.0 | 1.2 |
| Poly(ε-caprolactone), Mn ∼ 42500 | 51.0 | 5.1 |
| Polyethylene oxide (Polyox WSR 301) | 10.0 | 1.0 |
| Butylated Hydroxytoluene | 1.0 | 0.1 |
| Total | 74.0 | 7.4 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 3 minutes.
3. Melting and Blending: The polyethylene oxide (PEO301) was slowly added to the beaker containing the melted PCL/BHT and mixed on a Thermodyne Thermodyne Hot Plate (temperature range 90° - 160°C) until mixture appeared homogeneous.
4. Melting and Blending: The Naltrexone HCl was slowly added to the PCL/PEO/BHT and mixed on a Thermodyne Thermodyne Hot Plate (temperature range 90° - 160°C) until the mixture appeared homogeneous.
5. Casting: The molten drug/polymer blend was pressed between two stainless steel plates to a thickness of approximately 2 millimeters.
6. Cooling: The drug/polymer blend was cooled at room temperature.
7. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid with 0.1% Sodium Lauryl Sulfate or 900 ml Simulated Gastric Fluid with 40% ethanol.
4. Analytical Method - UV Analysis, UV/Vis Spectrophotometer setup with flow
5. through cells (wavelength 230 nm). Peristaltic pump (flow rate approx 5 ml/min).
6. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

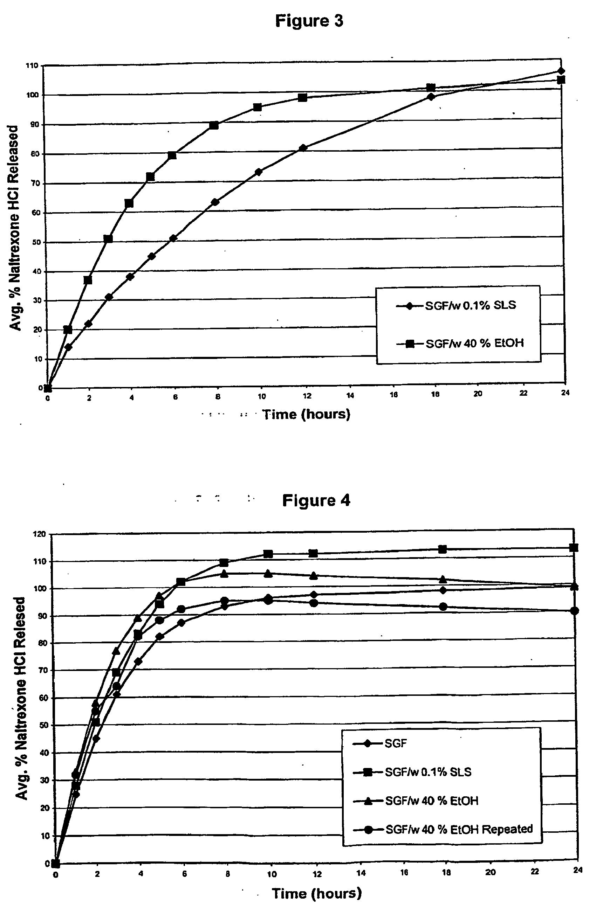

The dissolution results for the poly(ε-caprolactone) multiparticules are summarized in Figure 3 and Table 3a.

**Table 3a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF/w 0.1% SLS** | 14 | 22 | 31 | 38 | 45 | 51 | 63 | 73 | 81 | 98 | 106 |
| **SGF with 40% EtOH** | 20 | 37 | 51 | 63 | 72 | 79 | 89 | 95 | 98 | 101 | 103 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind and fused/melted during milling.

### REFERENCE EXAMPLE 4

The composition of the poly(ε-caprolactone) multiparticulates is summarized in Table 4 below.

**Table 4**

| **Ingredient (Trade Name)** | **Amt/unit (mg)** | **Amt/batch (g)** |
|---|---|---|
| Naltrexone HCl | 12.0 | 1.2 |
| Poly(ε-caprolactone), Mn ∼ 42500 | 72.0 | 7.2 |
| Polyethylene oxide (Polyox WSR 301) | 35.0 | 3.5 |
| Butylated Hydroxytoluene | 1.0 | 0.1 |
| Total | 120.0 | 12.0 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 5 minutes.
3. Melting and Blending: The polyethylene oxide (PEO301) was slowly added to the beaker containing the melted PCL/BHT and mixed on a Thermodyne Thermodyne Hot Plate (temperature range 90° - 160°C) until mixture appeared homogeneous.
4. Melting and Blending: The Naltrexone HCl was slowly added to the PCL/PEO/BHT and mixed on a Thermodyne Thermodyne Hot Plate (temperature range 90° - 160°C) until the mixture appeared homogeneous.
5. Casting: The molten drug/polymer blend was pressed between two stainless steel plates to a thickness of approximately 2 millimeters.
6. Cooling: The drug/polymer blend was cooled at room temperature.
7. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 720 minutes.
3. Media - 900 ml Simulated Gastric Fluid, Simulated Gastric Fluid with 0.1% Sodium Lauryl Sulfate or 900 ml Simulated Gastric Fluid with 40% ethanol.
4. Analytical Method - UV Analysis, UV/Vis Spectrophotometer setup with flow
5. through cells (wavelength 230 nm). Peristaltic pump (flow rate approx 5 ml/min).
6. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

The dissolution results for the poly(ε-caprolactone) multiparticules are summarized in Figure 4 and Table 4a.

**Table 4a**

| | **Dissolution Results** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 25 | 45 | 61 | 73 | 82 | 87 | 93 | 96 | 97 | 99 | 99 |
| **SGF/w 0.1% SLS** | 28 | 51 | 69 | 83 | 94 | 102 | 109 | 112 | 112 | 113 | 113 |
| **SGF with 40% EtOH** | 33 | 58 | 77 | 89 | 97 | 102 | 105 | 105 | 104 | 102 | 99 |
| **SGF with 40% EtOH Repeated** | 32 | 55 | 64 | 82 | 89 | 92 | 95 | 95 | 94 | 92 | 90 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind but did not fuse/melt during milling.

### REFERENCE EXAMPLE 5

The composition of this poly(ε-caprolactone) multiparticulate formulation is summarized in Table 5.

**Table 5**

| **Ingredient (Trade Name)** | **Amt/unit (mg)** | **Amt/unit (%)** | **Amt/Batch (g)** |
|---|---|---|---|
| Naltrexone HCl | 12.0 | 10.00 | 1.2 |
| Poly(ε-caprolactone), Mn ∼ 42500 | 97.0 | 80.83 | 9.7 |
| Poloxamer (Lutrol 68) | 10.0 | 8.33 | 1.0 |
| Butylated Hydroxytoluene | 1.0 | 0.83 | 0.1 |
| Total | 120.0 | 100.0 | 12.0 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 5 minutes.
3. Melting and Blending: The poloxamer (Lutrol 68) was slowly added to the beaker containing the melted PCL/BHT and mixed on a Thermodyne Thermodyne Hot Plate (temperature range 90° - 160°C) until mixture appeared homogeneous.
4. Melting and Blending: The Naltrexone HCl was slowly added to the PCL/Poloxamer/BHT and mixed on a Thermodyne Thermodyne Hot Plate (temperature range 90° - 160°C) until the mixture appeared homogeneous.
5. Casting: The molten drug/polymer blend was pressed between two stainless steel plates to a thickness of approximately 2 millimeters.
6. Cooling: The drug/polymer blend was cooled at room temperature.
7. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid or 900 ml Simulated Gastric Fluid with 40% ethanol.
4. Analytical Method - UV Analysis, UV/Vis Spectrophotometer setup with flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

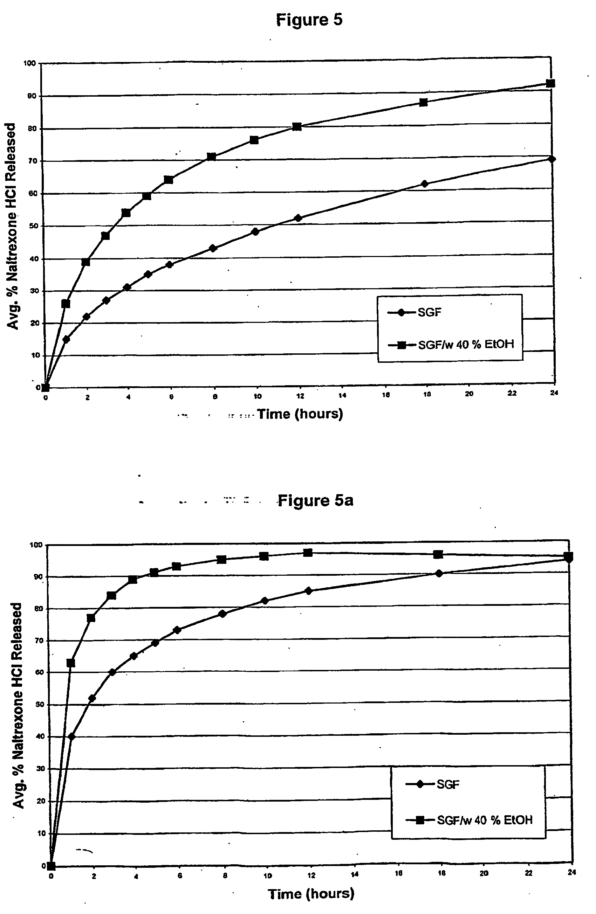

The dissolution results for the poly(ε-caprolactone) multiparticules are summarized in Figure 5 and Table 5a.

**Table 5a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 15 | 22 | 27 | 31 | 35 | 38 | 43 | 48 | 52 | 62 | 69 |
| **SGF with 40% EtOH** | 26 | 39 | 47 | 54 | 59 | 64 | 71 | 76 | 80 | 87 | 92 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The dissolution results for the milled poly(ε-caprolactone) multiparticules are summarized in Figure 5a and Table 5b.

The poly(ε-caprolactone) pellets were tough. The pellets did not fuse/melt during milling.

**Table 5b**

| | **Dissolution Result Milled** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 40 | 52 | 60 | 65 | 69 | 73 | 78 | 82 | 85 | 90 | 94 |
| **SGF with 40% EtOH** | 63 | 77 | 84 | 89 | 91 | 93 | 95 | 96 | 97 | 96 | 95 |

### REFERENCE EXAMPLE 6

The composition of this poly(ε-caprolactone) multiparticulate formulation is summarized in Table 6.

**Table 6**

| **Ingredient (Trade Name)** | **Amt/unit (mg)** | **Amt/unit (%)** | **Amt/Batch (g)** |
|---|---|---|---|
| Naltrexone HCl | 12.0 | 10.00 | 1.2 |
| Poly(ε-caprolactone), Mn ∼ 42,500 | 61.0 | 50.83 | 6.1 |
| Poly(ε-caprolactone), Mn ∼ 10,000 | 36.0 | 30.00 | 3.6 |
| Polyethylene oxide (Polyox WSR 301) | 10.0 | 8.33 | 1.0 |
| Butylated Hydroxytoluene | 1.0 | 0.83 | 0.1 |
| Total | 120.0 | 100.0 | 12.0 |

The processing steps for manufacturing the poly(ε-caprolactone) multiparticulates are as follows:
1. Milling: The butylated hydroxytoluene (BHT) was milled with a mortar and pestle.
2. Melting and Blending: The low moleculare weight poly(ε-caprolactone) (PCL), high moleculare weight polycaprolactne (PCL) and milled BHT were melted and mixed on a Thermodyne Thermodyne Hot Plate (temperature range 90° - 160°C) for approximately 5 minutes.
3. Melting and Blending: The polyethylene oxide (PEO301) was slowly added to the beaker containing the melted PCL/BHT and mixed on a Thermodyne Thermodyne Hot Plate (temperature range 90° - 160°C) until mixture appeared homogeneous.
4. Melting and Blending: The Naltrexone HCl was slowly added to the PCL/PEO/BHT and mixed on a Thermodyne Thermodyne Hot Plate (temperature range 90° - 160°C) until the mixture appeared homogeneous.
5. Casting: The molten drug/polymer blend was pressed between two stainless steel plates to a thickness of approximately 2 millimeters.
6. Cooling: The drug/polymer blend was cooled at room temperature.
7. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid, Simulated Gastric Fluid with 0.1 % Sodium Lauryl Sulfate or 900 ml Simulated Gastric Fluid with 40% ethanol.
4. Analytical Method - UV Analysis, UV/Vis Spectrophotometer setup with Flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

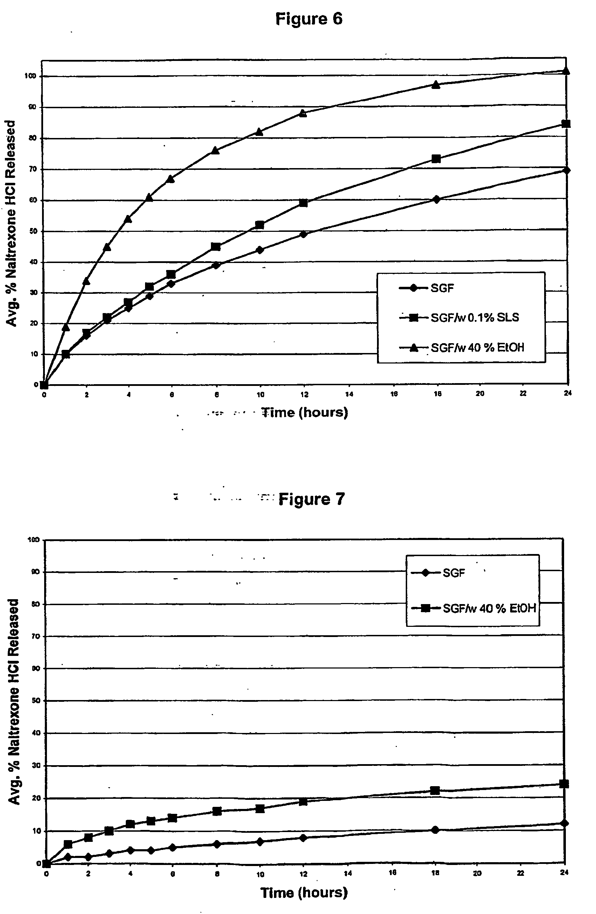

The dissolution results for the poly(ε-caprolactone) multiparticules are summarized in Figure 6 and Table 6a.

**Table 6a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 10 | 16 | 21 | 25 | 29 | 33 | 39 | 44 | 49 | 60 | 69 |
| **SGF/w 0.1% SLS** | 10 | 17 | 22 | 27 | 32 | 36 | 45 | 52 | 59 | 73 | 84 |
| **SGF with 40% EtOH** | 19 | 34 | 45 | 54 | 61 | 67 | 76 | 82 | 88 | 97 | 101 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were tough and difficult to grind. During milling the discrete matrix particles formed a single fused mass.

### REFERENCE EXAMPLE 7

The composition of this poly(ε-caprolactone) multiparticulate formulation is summarized in Table 7.

**Table 7**

| **Ingredient (Trade Name)** | **Amt/unit (mg)** | **Amt/unit (%)** | **Amt/Batch (g)** |
|---|---|---|---|
| Naltrexone HCl | 12.0 | 9.9 | 1.2 |
| Poly(ε-caprolactone), Mn ∼ 42500 | 108.0 | 89.3 | 10.8 |
| Butylated Hydroxytoluene | 1.0 | 0.8 | 0.1 |
| Total | 121.0 | 100.0 | 12.1 |

The following processing steps were used to manufacturing the poly(ε-caprolactone) multiparticulates.
1. Milling: The butylated hydroxytoluene (BHT) was milled with a mortar and pestle.
2. Melting and Blending: The poly(ε-caprolactone) and milled BHT were melted and mixed on a Thermodyne Thermodyne Hot Plate (temperature range 90° - 160°C) for approximately 3 minutes.
3. Melting and Blending: The Naltrexone HCl was slowly added to the polymer mixture and mixed on a Thermodyne Thermodyne Hot Plate (temperature range 90° - 160°C) until the blend appeared homogeneous.
4. Casting: The molten drug/polymer blend was pressed between two stainless steel plates to a thickness of approximately 3.
5. Cooling: The drug/polymer blend was cooled at room temperature.
6. Pelletizing: The drug/polymer sheet was cut into pellets approximately 3 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid (SGF), or Simulated Gastric Fluid with 40% ethanol (EtOH).
4. Analytical Method - UV Analysis, UV/Vis Spectrophotometer setup with flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

The dissolution results for the poly(ε-caprolactone) multiparticules are summarized in Figure 7 and Table 7a.

**Table 7a**

| | **Dissolution Result** | | | | | | | | | | |
|---|---|---|---|---|---|---|---|---|---|---|---|
| **Dissolution Media** | **Mean Naltrexone HCl % Released** | | | | | | | | | | |
| | 1 h | 2 h | 3 h | 4 h | 5 h | 6 h | 8 h | 10 h | 12 h | 18 h | 24 h |
| **SGF (n=6)** | 2 | 2 | 3 | 4 | 4 | 5 | 6 | 7 | 8 | 10 | 12 |
| **SGF with 40% EtOH (n=2)** | 6 | 8 | 10 | 12 | 13 | 14 | 16 | 17 | 19 | 22 | 24 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

The poly(ε-caprolactone) pellets were tough and fused/melted during milling.

### REFERENCE EXAMPLE 8

The composition of this poly(ε-caprolactone) multiparticulate formulation is summarized in Table 8.

**Table 8**

| **Ingredient (Trade Name)** | **Amt/unit (mg)** | **Amt/unit (%)** | **Amt/Batch (g)** |
|---|---|---|---|
| Naltrexone HCl | 12.0 | 12.77 | 2.4 |
| Poly(ε-caprolactone), Mn ∼ 42500 | 81.0 | 86.17 | 16.2 |
| Butylated Hydroxytoluene | 1.0 | 1.06 | 0.2 |
| Total | 94.0 | 100.00 | 18.8 |

The following processing steps were used to manufacturing the poly(ε-caprolactone) multiparticulates.
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 3 minutes.
3. Melting and Blending: The Naltrexone HCl was slowly added to the polymer mixture and mixed on a Thermodyne Thermodyne Hot Plate (temperature range 90° - 160°C) until the blend appeared homogeneous.
4. Casting: The molten drug/polymer blend was pressed between two stainless steel plates to a thickness of approximately 2 millimeters.
5. Cooling: The drug/polymer blend was cooled at room temperature.
6. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid (SGF) with 0.5% Sodium Lauryl Sulfate (SLS), Simulated Gastric Fluid (SGF) with 0.1% Sodium Lauryl Sulfate (SLS) or Simulated Gastric Fluid with 40% ethanol (EtOH).
4. Analytical Method - UV Analysis, UV/Vis Spectrophotometer setup with flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator.

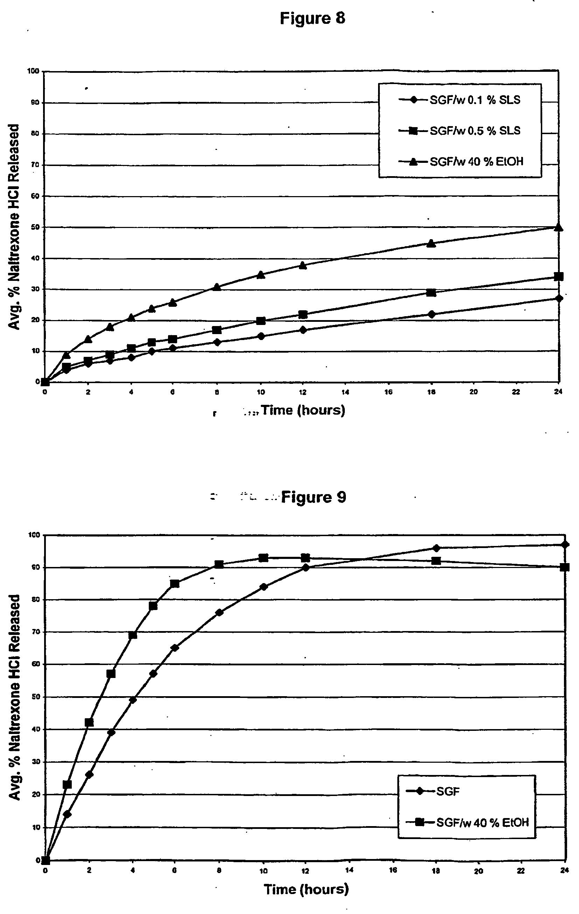

The dissolution results for the poly(ε-caprolactone) multiparticulates are summarized in Figure 8 and Table 8a.

**Table 8a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF/w 0.1% SLS** | 4 | 6 | 7 | 8 | 10 | 11 | 13 | 15 | 17 | 23 | 27 |
| **SGF/w 0.5% SLS** | 5 | 7 | 9 | 11 | 13 | 14 | 17 | 20 | 22 | 29 | 34 |
| **SGF with 40% EtOH** | 9 | 14 | 18 | 21 | 24 | 26 | 31 | 35 | 38 | 45 | 50 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind and fused/melted during milling.

### REFERENCE EXAMPLE 9

The composition of this poly(ε-caprolactone) multiparticulate formulation is summarized in Table 9.

**Table 9**

| **Ingredient (Trade Name)** | **Amt/unit (mg)** | **Amt/unit (%)** | **Amt/Batch (g)** |
|---|---|---|---|
| Naltrexone HCl | 12.0 | 10.00 | 1.2 |
| Poly(ε-caprolactone), Mn ∼ 42500 | 82.0 | 68.33 | 8.2 |
| Polyethylene oxide (Polyox WSR 301) | 25.0 | 20.83 | 2.5 |
| Butylated Hydroxytoluene | 1.0 | 0.83 | 0.1 |
| Total | 120.0 | 100.0 | 12.0 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 5 minutes.
3. Melting and Blending: The polyethylene oxide (PEO301) was slowly added to the beaker containing the melted PCL/BHT and mixed on a Thermodyne Thermodyne Hot Plate (temperature range 90° - 160°C) until mixture appeared homogeneous.
4. Melting and Blending: The Naltrexone HCl was slowly added to the PCL/PEO/BHT and mixed on a Thermodyne Thermodyne Hot Plate (temperature range 90° - 160°C) until the mixture appeared homogeneous.
5. Casting: The molten drug/polymer blend was pressed between two stainless steel plates to a thickness of approximately 2 millimeters.
6. Cooling: The drug/polymer blend was cooled at room temperature.
7. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid or 900 ml Simulated Gastric Fluid with 40% ethanol.
4. Analytical Method - UV Analysis, UV/Vis Spectrophotometer setup with flow
5. through cells (wavelength 230 nm). Peristaltic pump (flow rate approx 5 ml/min).
6. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

The dissolution results for the poly(ε-caprolactone) multiparticules are summarized in Figure 9 and Table 9a.

**Table 9a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 14 | 26 | 39 | 49 | 57 | 64 | 76 | 84 | 90 | 96 | 97 |
| **SGF with 40% EtOH** | 23 | 42 | 57 | 69 | 78 | 85 | 91 | 93 | 93 | 92 | 90 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind and fused/melted during milling.

### REFERENCE EXAMPLE 10

The composition of this poly(ε-caprolactone) multiparticulate formulation is summarized in Table 10.

**Table 10**

| **Ingredient (Trade Name)** | **Amt/unit (mg)** | **Amt/unit (%)** | **Amt/Batch (g)** |
|---|---|---|---|
| Naltrexone HCl | 12.0 | 10.00 | 1.2 |
| Poly(ε-caprolactone), Mn ∼ 42500 | 72.0 | 60.00 | 7.2 |
| Polyethylene oxide (Polyox WSR 303) | 25.0 | 20.83 | 2.5 |
| Polyethylene Glycol 3350 (Carbowax Sentry PEG 3350) | 10.0 | 8.33 | 1.0 |
| Butylated Hydroxytoluene | 1.0 | 0.83 | 0.1 |
| Total | 120.0 | 100.0 | 12.0 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 5 minutes.
3. Melting and Blending: The polyethylene Glycol (PEG 3350) was slowly added to the beaker containing the melted PCL/BHT and mixed on a Thermodyne Thermodyne Hot Plate (temperature range 90° - 160°C) until mixture appeared homogeneous.
4. Melting and Blending: The polyethylene oxide (PEO301) was slowly added to the beaker containing the melted PCL/PEG/BHT and mixed on a Thermodyne Thermodyne Hot Plate (temperature range 90° - 160°C) until mixture appeared homogeneous.
5. Melting and Blending: The Naltrexone HCl was slowly added to the PCL/PEO/BHT and mixed on a Thermodyne Thermodyne Hot Plate (temperature range 90° - 160°C) until the mixture appeared homogeneous.
6. Casting: The molten drug/polymer blend was pressed between two stainless steel plates to a thickness of approximately 2 millimeters.
7. Cooling: The drug/polymer blend was cooled at room temperature.
8. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid, Simulated Gastric Fluid with 0.1% Sodium Lauryl Sulfate or 900 ml Simulated Gastric Fluid with 40% ethanol.
4. Analytical Method - UV Analysis, UV/Vis Spectrophotometer setup with flow
5. through cells (wavelength 230 nm). Peristaltic pump (flow rate approx 5 ml/min).
6. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

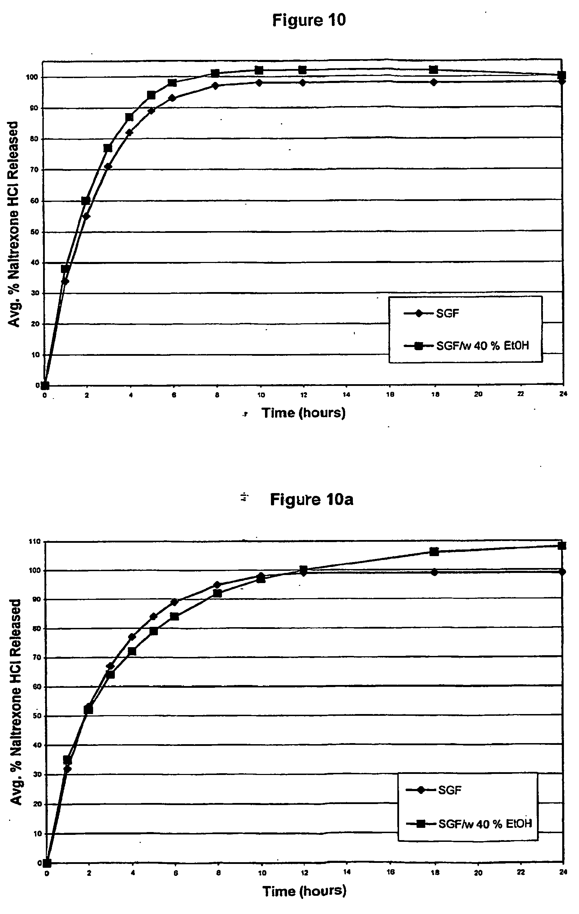

The dissolution results for the poly(ε-caprolactone) multiparticules are summarized in Figure 10 and Table 10a.

**Table 10a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 34 | 55 | 71 | 82 | 89 | 93 | 97 | 98 | 98 | 98 | 98 |
| **SGF with 40% EtOH** | 38 | 60 | 77 | 87 | 94 | 98 | 101 | 102 | 102 | 102 | 100 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to grind and fused/melted during milling. The dissolution results for milled samples are summarized in Figure 10a and Table 10b.

**Table 10b**

| | **Dissolution Result Milled** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 32 | 53 | 67 | 77 | 84 | 89 | 95 | 98 | 99 | 99 | 99 |
| **SGF with 40% EtOH** | 35 | 52 | 64 | 72 | 79 | 84 | 92 | 97 | 100 | 106 | 108 |

### REFERENCE EXAMPLE 11

The composition of this poly(ε-caprolactone) multiparticulate formulation is summarized in Table 11.

**Table 11**

| **Ingredient (Trade Name)** | **Amt/unit (mg)** | **Amt/unit (%)** | **Amt/Batch (g)** |
|---|---|---|---|
| Naltrexone HCl | 12.0 | 10.00 | 1.2 |
| Poly(ε-caprolactone), Mn ∼ 10,000 | 82.0 | 68.33 | 8.2 |
| Polyethylene oxide (Polyox WSR 301) | 25.0 | 20.83 | 2.5 |
| Butylated Hydroxytoluene | 1.0 | 0.83 | 0.1 |
| Total | 120.0 | 100.0 | 12.0 |

The processing steps for manufacturing the poly(ε-caprolactone) multiparticulates are as follows:
1. Milling: The butylated hydroxytoluene (BHT) was milled with a mortar and pestle.
2. Melting and Blending: The poly(ε-caprolactone) (PCL) and milled BHT were melted and mixed on a Thermodyne Thermodyne Hot Plate (temperature range 90° - 160°C) for approximately 5 minutes.
3. Melting and Blending: The polyethylene oxide (PEO301) was slowly added to the beaker containing the melted PCL/BHT and mixed on a Thermodyne Thermodyne Hot Plate (temperature range 90° - 160°C) until mixture appeared homogeneous.
4. Melting and Blending: The Naltrexone HCl was slowly added to the PCL/PEO/BHT and mixed on a Thermodyne Hot Plate (temperature range 90° - 160°C) until the mixture appeared homogeneous.
5. Casting: The molten drug/polymer blend was pressed between two stainless steel plates to a thickness of approximately 2 millimeters.
6. Cooling: The drug/polymer blend was cooled at room temperature.
7. Pelletizing: The drug/polymer sheet was cut into pellets approximately 2 mm in length and width.

### Dissolution Method

1. Apparatus- USP Type I (Baskets), 100 rpm at 37°C.
2. Sampling time - every minute up to 1440 minutes.
3. Media - 900 ml Simulated Gastric Fluid or 900 ml Simulated Gastric Fluid with 40% ethanol.
4. Analytical Method - UV Analysis, UV/Vis Spectrophotometer setup with flow
5. through cells (wavelength 230 nm). Peristaltic pump (flow rate approx 5 ml/min).
6. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

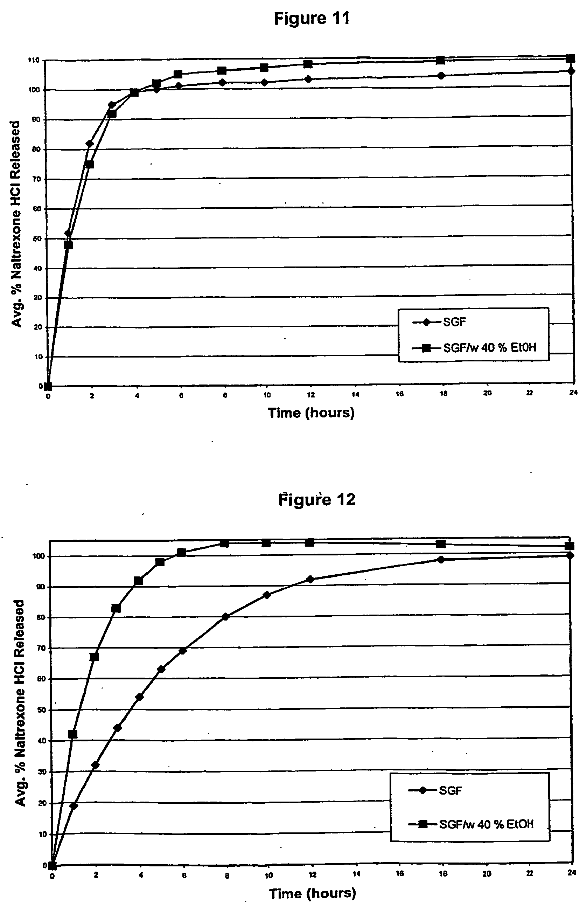

The dissolution results for the poly(ε-caprolactone) multiparticules are summarized in Figure 11 and Table 11a.

**Table 11a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 52 | 82 | 95 | 99 | 100 | 101 | 102 | 102 | 103 | 104 | 105 |
| **SGF with 40% EtOH** | 48 | 75 | 92 | 99 | 102 | 105 | 106 | 107 | 108 | 109 | 109 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

The poly(ε-caprolactone) pellets were waxy and brittle. They did not fuse/melt during milling.

### REFERENCE EXAMPLE 12

The composition of the poly(ε-caprolactone) melt extruded multiparticulates is summarized in Table 12 below.

**Table 12**

| **Ingredient (Trade Name)** | **Amt/unit (mg)** | **Amt/batch (g)** |
|---|---|---|
| Naltrexone HCl | 12.0 | 200.00* |
| Poly(ε-caprolactone) Mw ∼ 42500 | 97.0 | 1,616.67 |
| Polyethylene oxide (Polyox WRS 301) | 10.0 | 166.67 |
| Butylated hydroxytoluene (BHT), Milled | 1.0 | 16.67 |
| Total | 120.0 | 2000.0 |

| | | |
|---|---|---|
| * Weigh is not corrected for water or impurities | | |

The processing conditions at the time of sampling are summarized below.

### Extruder: Leistritz ZSE 27

Screw Configuration: Counter-rotation

| | | | | | | | | | | | | |
|---|---|---|---|---|---|---|---|---|---|---|---|---|
| Heating Zone | 1 | 2 | 3 | 4 | 5 | 6 | 7 | 8 | 9 | 10 | 11 | 12 |
| Temperature (°C) | 18 | 36 | 66 | 78 | 78 | 78 | 77 | 76 | 77 | 80 | 88 | 76 |

Torque (%): 97
Melt Pressure (psi): 1930
Feed rate (g/min.): 20
Screw speed (rpm): 66
Die Plate Hole diameter (mm): 1.0 (8-hole die plate)

### Equipment

Leistritz - ZSE 27 Twin Screw Extruder (Counter-Rotation)
Neslab Model CFT-150 Chiller
Accurate Powder Feeder
Dorner 8-foot Conveyor
Grablab Electronic Timer

The processing steps for manufacturing Poly(ε-caprolactone) melt extruded multiparticulates are as follows:
1. Screening: Naltrexone HCl, Poly(ε-caprolactone), Polyethylene oxide and BHT were screened through a # 20 US mesh screen.
2. Blending: The materials screened in Step 1 were loaded into an 8 qt. V-blender (½ Poly(ε-caprolactone), Naltrexone HCl, Polyethylene oxide, BHT and ½ Poly(ε-caprolactone)) and blended for 10 minutes at ambient temperature.
3. Extrusion: Materials blended in Step 2 were metered into a twin screw extruder fitted with a die and processed into approximately 1 mm strands. The extruder was set on counter-rotation with zone (barrel) temperatures ranged from 18°C to 88°C.
4. Cooling: The strands were cooled on a conveyor at ambient temperature.
5. Pelletizing: The cooled strands were cut into pellets approximately 1 mm in length.

### Dissolution Method

The following method was used to test the dissolution of the poly(ε-caprolactone) multiparticulates.
1. Apparatus- USP Type I (Baskets), 100 rpm at 37°C.
2. Sampling time - every minute up to 1440 minutes.
3. Media - 900 ml Simulated Gastric Fluid (SGF) or Simulated Gastric Fluid with 40% ethanol (EtOH).
4. Analytical Method - UV Analysis, UV/Vis Spectrophotometer setup with flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

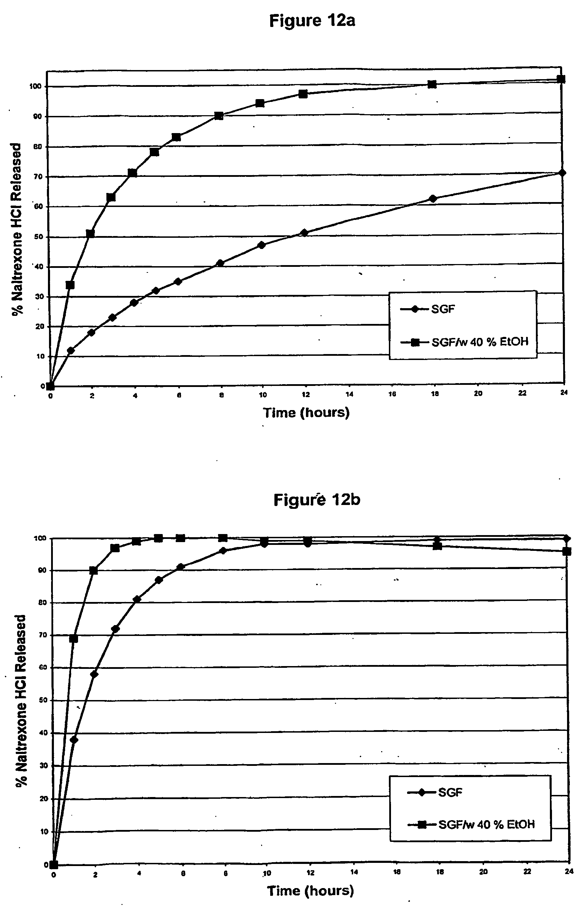

The dissolution results for the poly(ε-caprolactone) multiparticules are summarized in Figure 12 and Table 12a.

**Table 12a**

| | **Dissolution Result** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 19 | 32 | 44 | 54 | 63 | 69 | 80 | 87 | 92 | 98 | 99 |
| **SGF with 40% EtOH** | 42 | 67 | 83 | 92 | 98 | 101 | 104 | 104 | 104 | 103 | 102 |

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

The poly(ε-caprolactone) pellets were difficult to crush with a mortar and pestle. They fused/melted during milling but incomplete after 15 seconds.

The dissolution results for the ground (Figure 12a and Table 12b) and milled (Figure 12b and Table 12c) poly(ε-caprolactone) pellets are summarized below.

**Table 12b**

| | **Dissolution Result Ground** | | | | | | | | | | |
|---|---|---|---|---|---|---|---|---|---|---|---|
| **Dissolution Media** | **Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 12 | 18 | 23 | 28 | 32 | 35 | 41 | 47 | 51 | 62 | 70 |
| **SGF with 40% EtOH** | 34 | 51 | 63 | 71 | 78 | 83 | 90 | 94 | 97 | 100 | 101 |

**Table 12c**

| | **Dissolution Result Milled** | | | | | | | | | | |
|---|---|---|---|---|---|---|---|---|---|---|---|
| **Dissolution Media** | **Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 38 | 58 | 72 | 81 | 87 | 91 | 96 | 98 | 98 | 99 | 99 |
| **SGF with 40% EtOH** | 69 | 90 | 97 | 99 | 100 | 100 | 100 | 99 | 99 | 97 | 95 |

### REFERENCE EXAMPLE 13

The composition of the Poly(ε-caprolactone) melt extruded multiparticulates is summarized in Table 13 below.

**Table 13**

| **Ingredient (Trade Name)** | **Amt/unit (mg)** | **Amt/batch (g)** |
|---|---|---|
| Naltrexone HCl | 12.0 | 150.0* |
| Poly(ε-caprolactone) Mw ∼ 42500 | 97.0 | 1,212.5 |
| Polyethylene oxide (Polyox WRS 303) | 7.0 | 87.5 |
| Polyethylene Glycol (PEG 3350) | 3.0 | 37.5 |
| Butylated hydroxytoluene (BHT), Milled | 1.0 | 12.5 |
| Total | 120.0 | 1500.0 |

| | | |
|---|---|---|
| * Weigh is not corrected for water or impurities | | |

The processing conditions at the time of sampling are summarized below.
Extruder: Leistritz ZSE 27
Screw Configuration: Counter-rotation

### Sample 1 mm Strands

| | | | | | | | | | | | | |
|---|---|---|---|---|---|---|---|---|---|---|---|---|
| Heating Zone | 1 | 2 | 3 | 4 | 5 | 6 | 7 | 8 | 9 | 10 | 11 | 12 |
| Temperature (°C) | 46 | 54 | 75 | 90 | 90 | 90 | 94 | 96 | 92 | 89 | 90 | 88 |

Torque (%): 53
Melt Pressure (psi): 890
Feed rate (g/min.): 11
Screw speed (rpm): 20
Melt Temp. (°C): 94
Die Plate Hole diameter (mm): 1.0 (8-hole die plate)

### Sample 1.5 mm Strands

| | | | | | | | | | | | | |
|---|---|---|---|---|---|---|---|---|---|---|---|---|
| Heating Zone | 1 | 2 | 3 | 4 | 5 | 6 | 7 | 8 | 9 | 10 | 11 | 12 |
| Temperature (°C) | 45 | 53 | 74 | 90 | 89 | 91 | 93 | 89 | 90 | 90 | 89 | 89 |

Torque (%): 55
Melt Pressure (psi): 870
Feed rate (g/min.): 11
Screw speed (rpm): 20
Melt Temp. (°C): 93
Die Plate Hole diameter (mm): 1.0 (8-hole die plate)

### Sample 2 mm Strands

| | | | | | | | | | | | | |
|---|---|---|---|---|---|---|---|---|---|---|---|---|
| Heating Zone | 1 | 2 | 3 | 4 | 5 | 6 | 7 | 8 | 9 | 10 | 11 | 12 |
| Temperature | 48 | 60 | 87 | 95 | 97 | 10 | 11 | 10 | 93 | 95 | 80 | 81 |
| (°C) | | | | | | 3 | 0 | 3 | | | | |

Torque (%): 62
Melt Pressure (psi): 370
Feed rate (g/min.): 22
Screw speed (rpm): 50
Melt Temp. (°C): 89
Die Plate Hole diameter (mm): 3.0 (10-hole die plate)

### Equipmet

Leistritz - ZSE 27 Twin Screw Extruder (Counter-Rotation)
Neslab Model CFT-150 Chiller
Accurate Powder Feeder
Domer 8-foot Conveyor
Grablab Electronic Timer

The processing steps for manufacturing Poly(ε-caprolactone) melt extruded multiparticulates are as follows:
1. Screening: Naltrexone HCl, Poly(ε-caprolactone), Polyethylene Glycol and BHT were screened through a # 20 US mesh screen. Polyethylene oxide screened through a #100 US mesh screen.
2. Blending: The materials screened in Step 1 were loaded into an 8 qt. V-blender (½ Poly(ε-caprolactone), Naltrexone HCl, Polyethylene oxide, polyethylene glycol, BHT and ½ Poly(ε-caprolactone)) and blended for 10 minutes at ambient temperature.
3. Extrusion: Materials blended in Step 2 were metered into a twin screw extruder fitted with a die and processed into strands. The extruder was set on counter-rotation with zone (barrel) temperatures ranged from 18°C to 110°C.
4. Cooling: The strands were cooled on a conveyor at ambient temperature.
5. Pelletizing: The cooled strands were cut into pellets approximately 1.0 mm, 1.5 mm and 2.0 mm in length for Sample #3, Sample #4 and Sample #1, respectively.

### Dissolution Method

1. Apparatus- USP Type I (Baskets), 100 rpm at 37°C.
2. Sampling time - every minute up to 1440 minutes.
3. Media - 900 ml Simulated Gastric Fluid (SGF) or Simulated Gastric Fluid with 40% ethanol (EtOH).
4. Analytical Method - UV Analysis, UV/Vis Spectrophotometer setup with flow through cells (wavelength 230 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

### Milling Procedure

Equipment: IKA A11 Basic Impact Mill
Number of doses: 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W

### Grinding Procedure

Equipment: 8 oz Glass Mortar with Pestle
Number of doses: 2
Duration of grinding: 20 rotations

### Results

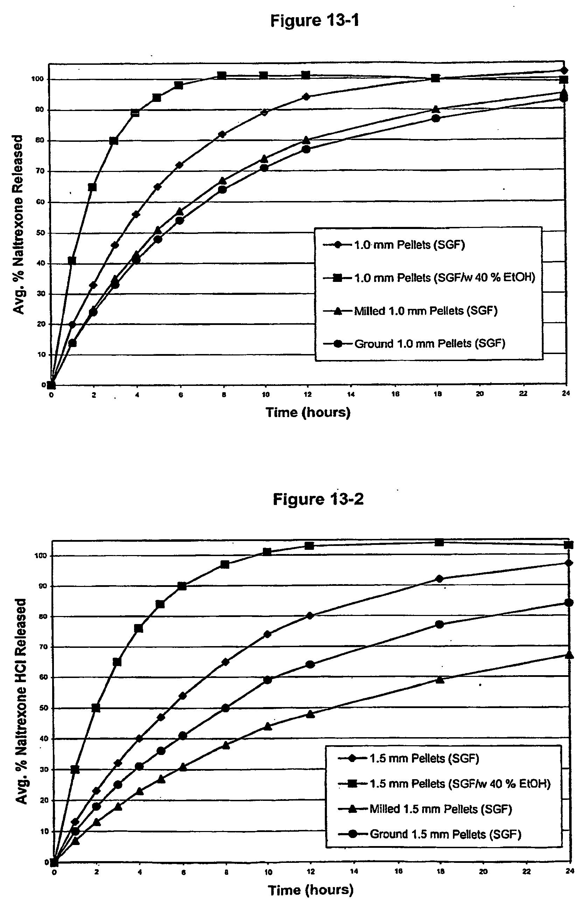

The dissolution results for the 1.0 mm (Table 13-1a, Figure 13-1), 1.5 mm (Table 13-2a, Figure 13-2) and 2.0 mm (Table 13-3a, Figure 13-3) poly(ε-caprolactone) pellets are summarized below.

The 1.0 mm, 1.5 mm and 2.0 mm poly(ε-caprolactone) pellets were difficult to grind with a mortar and pestle. All pellet samples fused/melted during milling. Dissolution results for the milled and ground pellets are summarized for the 1.0 mm (Figure 13-1 and Table 13-1b and c), 1.5 mm (Figure 13-2 and Table 13-2b and c) and 2.0 mm (Figure 13-3 and Table 13-3b and c) below.

**Table 13-1a**

| | **1.0 mm Pellets** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 20 | 33 | 46 | 56 | 65 | 72 | 82 | 89 | 94 | 100 | 102 |
| **SGF with 40% EtOH** | 41 | 65 | 80 | 89 | 94 | 98 | 101 | 101 | 101 | 100 | 99 |

**Table 13-1b**

| | **1.0 mm Pellets, milled** | | | | | | | | | | |
|---|---|---|---|---|---|---|---|---|---|---|---|
| **Dissolution Media** | **Mean Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 14 | 25 | 35 | 43 | 51 | 57 | 67 | 74 | 80 | 90 | 95 |

**Table 13-1c**

| | **1.0 mm Pellets, ground** | | | | | | | | | | |
|---|---|---|---|---|---|---|---|---|---|---|---|
| **Dissolution Media** | **Mean Naltrexone HCl % Released (n=1)** | | | | | | | | | | |
| | 1 h | 2 h | 3 h | 4 h | 5 h | 6 h | 8 h | 10 h | 12 h | 18 h | 24 |
| **SGF** | 14 | 24 | 33 | 41 | 48 | 54 | 64 | 71 | 77 | 87 | 93 |

**Table 13-2a**

| | **1.5 mm Pellets** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 13 | 23 | 32 | 40 | 47 | 54 | 65 | 74 | 80 | 92 | 97 |
| **SGF with 40% EtOH** | 30 | 50 | 65 | 76 | 84 | 90 | 97 | 101 | 103 | 104 | 103 |

**Table 13-2b**

| | **1.5 mm Pellets, milled** | | | | | | | | | | |
|---|---|---|---|---|---|---|---|---|---|---|---|
| **Dissolution Media** | **Mean Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 7 | 13 | 18 | 23 | 27 | 31 | 38 | 44 | 48 | 59 | 67 |

**Table 13-2c**

| | **1.5 mm Pellets, ground** | | | | | | | | | | |
|---|---|---|---|---|---|---|---|---|---|---|---|
| **Dissolution Media** | **Mean Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 10 | 18 | 25 | 31 | 36 | 41 | 50 | 58 | 64 | 77 | 84 |

**Table 13-3a**

| | **2.0 mm Pellets** | | | | | | | | | | |
|---|---|---|---|---|---|---|---|---|---|---|---|
| **Dissolution Media** | **Mean Naltrexone HCl % Released (n=2)** | | | | | | | | | | |
| | 1 h | 2 h | 3 h | 4 h | 5 h | 6 h | 8 h | 10 h | 12 h | 18 h | 24 h |
| **SGF** | 11 | 16 | 21 | 26 | 30 | 34 | 41 | 48 | 53 | 68 | 78 |
| **SGF with 40% EtOH** | 22 | 36 | 46 | 55 | 62 | 69 | 78 | 85 | 90 | 97 | 99 |

**Table 13-3b**

| | **2.0 mm Pellets, milled** | | | | | | | | | | |
|---|---|---|---|---|---|---|---|---|---|---|---|
| **Dissolution Media** | **Mean Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 5 | 8 | 11 | 13 | 15 | 17 | 21 | 24 | 27 | 34 | 40 |

**Table 13-3c**

| | **2.0 mm Pellets, ground** | | | | | | | | | | |
|---|---|---|---|---|---|---|---|---|---|---|---|
| **Dissolution Media** | **Mean Naltrexone HCl % Released (n=1)** | | | | | | | | | | |
| | 1 h | 2 h | 3 h | 4 h | 5 h | 6 h | 8 h | 10 h | 12 h | 18 h | 24 h |
| **SGF** | 13 | 19 | 25 | 29 | 33 | 37 | 44 | 49 | 55 | 67 | 76 |

Futher Equipment used in the Examples
Mettler, Sartorious balances
Starrett Micrometers
Fluka Digital Thermometer
Carver Model 4332 Press

### EXAMPLE 14

The composition of the Poly(ε-caprolactone) melt extruded multiparticulates/pellets is summarized in Table 14 below.

**Table 14**

| **Ingredient (Trade Name)** | **Amt/unit (mg)** | **Amt/batch (g)** |
|---|---|---|
| Oxycodone HCl | 20.0* | 750.00* |
| Poly(ε-caprolactone) Mw ∼ 42500 | 101.1 | 3791.66 |
| Polyethylene oxide (Polyox WRS 303) | 7.8 | 291.66 |
| Polyethylene Glycol (PEG 3350) | 3.3 | 125.00 |
| Butylated hydroxytoluene (BHT), Milled | 1.1 | 41.66 |
| Total | 133.3 | 5000.0 |

| | | |
|---|---|---|
| * Weigh is not corrected for water or impurities | | |

The processing conditions at the time of sampling are summarized below.
Extruder: Leistritz ZSE 27
Screw Configuration: Counter-rotation

| | | | | | | | | | | | | |
|---|---|---|---|---|---|---|---|---|---|---|---|---|
| Heating Zone | 1 | 2 | 3 | 4 | 5 | 6 | 7 | 8 | 9 | 10 | 11 | 12 |
| Temperature (°C) | 14-18 | 35-50 | 65-75 | 90 | 90 | 90 | 90 | 90 | 90 | 90 | 89-91 | 85-90 |

Torque (%): 57-67
Melt Pressure (psi): 230-270
Feed rate (g/min.): 20-22
Screw speed (rpm): 20
Melt Temp. (°C): 93-96
Die Plate Hole diameter (mm): 3.0 (10-hole die plate)
Strand diameter: approximately 1.5 mm

### Equipment

Leistritz ZSE 27 Twin Screw Extruder (Counter-Rotation)
Neslab Model CFT-150 Chiller
Accurate Powder Feeder
Domer 8-foot Conveyor
Grablab Electronic Timer
Lasermike
Randcastle Pelletizer

The processing steps for manufacturing Poly(ε-caprolactone) melt extruded multiparticulates/pellets are as follows:
1. Screening Oxycodone HCl, Poly(ε-caprolactone) and BHT were screened through a # 20 US mesh screen. Polyethylene Glycol was screened through a #60 US mesh screen. Polyethylene oxide was screened through a #100 US mesh screen.
2. Blending: The materials screened in Step 1 were loaded into a 16 qt. V-blender (½ Poly(ε-caprolactone), Oxycodone HCl, Polyethylene oxide, polyethylene glycol, BHT and ½ Poly(ε-caprolactone)) and blended for 10 minutes at ambient temperature.
3. Extrusion: Materials blended in Step 2 were metered into a twin screw extruder fitted with a die and processed into strands. The extruder was set on counter-rotation with zone (barrel) temperatures ranged from 14°C to 90°C.
4. Cooling: The strands were cooled on a conveyor at ambient temperature.
5. Pelletizing: The cooled strands were cut into pellets approximately 1.5 mm in length.

### Dissolution Method I

1. Apparatus- USP Type I (Baskets), 100 rpm at 37°C.
2. Sampling time - every minute up to 1440 minutes.
3. Media - 900 ml Simulated Gastric Fluid or Simulated Gastric Fluid with 40% ethanol (EtOH).
4. Analytical Method - UV Analysis, UV/Vis Spectrophotometer setup with flow through cells (wavelength 240 nm). Peristaltic pump (flow rate approx 5 ml/min).
5. Equipment
   Perkin-Elmer Lambda 20 UV/Vis Spectrophotometer (8 - Position Cell Changer and Dissolution Manifold with tubing/connectors)
   Gilson Minipuls3 Peristaltic Pump
   Hellma 10 mm Quarts Flow Cells
   Perkin-Elmer UV WinLab Software/Microsoft Window 95 and Excel
   Hewlett-Packard Pavilion Computer Model 8240
   Van Kel VK 7010 Dissolution Bath (Fitted with Baskets)
   Van Kel VK 750D Heater/Circulator
   Branson 8510 Sonicator

### Milling Procedure

Equipment: IKA A11 Basic Impact Mill
Number of doses: Approximately 2
Duration of Milling: 15 seconds
Milling Chamber: Stainless steel
Chamber Volume: 80 ml
Blade: Stainless steel beater 1.4034
Rotor Shaft: Stainless steel 1.4571
Motor Speed, idle: 28000 revolutions/minute
Motor Speed, under load: 25000 revolutions/minute
Circumferential Speed, idle: 76 m/s
Circumferential Speed, under load: 53 m/s
Motor rating input: 160 W
Motor rating output: 100 W.

### Milling Procedure (Coffee Mill)

Equipment: Cuisinart Model DCG-12BC (120V, 60Hz, 12W)
Number of units: Approximately 2 units for pellets, 1 unit for tablet (comparison)
Duration of Milling: 60 seconds

### Grinding Procedure

Equipment: 8 oz Glass Mortar with Pestle
Number of doses: 2
Duration of grinding: 20 rotations

The dissolution results are summarized below in table 14-1a to c.

The poly(ε-caprolactone) pellets were difficult to grind with a mortar and pestle. All pellet samples fused/melted during milling. Dissolution results for the intact (Table 14-1a), milled (Table 14-1b) and ground (Table 14-lc) pellets are summarized below. Figure 14-2 depicts the a) intact, b) milled and c) ground pellets. Figure 14-3 depicts the a) the example pellets milled in a coffee mill and b) a comparison tablet without poly(ε-caprolactone) milled in a coffee mill. The composition and preparation of the comparison tablet without poly(ε-caprolactone) can be found in WO 2008/023261 Example 14.5.

**Table 14-1a**

| | **Intact** | | | | | | | | | | |
|---|---|---|---|---|---|---|---|---|---|---|---|
| **Dissol ution Media** | **Mean % Released (n=3)** | | | | | | | | | | |
| | 1 h | 2 h | 3 h | 4 h | 5 h | 6 h | 8 h | 10 h | 12 h | 18 h | 24 h |
| **SGF** | 16.1 | 25.0 | 32.9 | 40.1 | 46.7 | 52.7 | 62.9 | 71.1 | 77.8 | 91.2 | 97.8 |
| **SGF with 40% EtOH** | 32.0 | 49.0 | 62.2 | 71.9 | 79.0 | 84.2 | 91.2 | 95.2 | 97.5 | 99.2 | 99.0 |

**Table 14-1b**

| | **Milled** | | | | | | | | | | |
|---|---|---|---|---|---|---|---|---|---|---|---|
| **Dissolution Media** | **Mean % Released (n=3)** | | | | | | | | | | |
| | 1 h | 2 h | 3 h | 4 h | 5 h | 6 h | 8 h | 10 h | 12 h | 18 h | 24 h |
| **SGF** | 5.1 | 7.9 | 10.2 | 12.3 | 14.0 | 15.7 | 18.7 | 21.4 | 239 | 30.1 | 35.3 |
| **SGF with 40% EtOH** | 12.1 | 19.7 | 25.5 | 30.3 | 34.4 | 37.9 | 44.0 | 49.0 | 532 | 63.0 | 69.7 |

**Table 14-1c**

| | **Ground** | | | | | | | | | | |
|---|---|---|---|---|---|---|---|---|---|---|---|
| **Dissolution Media** | **Mean % Released (n=3)** | | | | | | | | | | |
| | 1 h | 2 h | 3 h | 4 h | 5 h | 6 h | 8 h | 10 h | 12 h | 18 h | 24 h |
| **SGF** | 13.7 | 21.0 | 26.5 | 31.2 | 35.1 | 39.2 | 45.7 | 51.3 | 56.1 | 67.3 | 75.7 |

### Stability Testing

The 1.5 mm pellets were placed on stability at 25°C/60% relative humidity (RH) and 40°C/75% RH in induction sealed high density polyethylene bottles (HDPE) with and without desiccant.

### Assay Method

The following method was used to assay the multiparticulates described in the example.
1. Extraction Solvent: 1:2 mixture acetonitrile and simulated gastric fluid without enzymes (SGF).
2. Analytical Method: Reversed-phase high performance liquid chromatography (HPLC) on a Waters Atlantis dC18 3.0 x 250 mm, 5 µm column maintained at 60°C using a mobile phase consisting of acetonitrile and potassium phosphate monobasic buffer at pH 3.0 with UV detection at 280 nm. Flow rate 1.0 ml/minute.
3. Equipment
   Waters Alliance 2695 HPLC system with 2487 UV-Visible absorbance detector
   Stir Plate

### Degradation Products Method

The following method was used to determine the degradation products of oxycodone HCl in the multiparticulates described in the example. Oxycodone N-oxide is the only known degradation product included in the % total degradation products. Noroxymorphone, oxymorphone, 10-hydroxyoxycodone, 6-α-oxycodol, 7,8-hydro-8, 14-dihydroxycodeinone, and hydrocodone which are known process impurities can be identified with this method but are not included in the calculation of % total degradation products.
1. Extraction Solvent: 1:2 mixture acetonitrile and simulated gastric fluid without enzymes (SGF).
2. Analytical Method: Reversed-phase high performance liquid chromatography (HPLC) on a YMC-Pack ODS-AQ 4.6 x 250 mm, 3 µm column maintained at 60°C using a mobile phase consisting of acetonitrile and potassium phosphate monobasic buffer at pH 3.0 with UV detection at 206 nm. Flow rate 1.0 ml/min.
3. Equipment
   Waters Alliance 2695 HPLC system with 2487 UV-Visible absorbance detector
   Waters Empower Software
   Stir Plate

### Dissolution Method II

The following method was used to test the dissolution of the multiparticulate stability samples described in the example.
1. Apparatus- USP Type I (Baskets), 100 rpm at 37°C.
2. Sampling Time- Generally, 1 hour, 2 hrs., 4 hrs., 8hrs., 12 hrs., 18 hrs. and 24 hrs.
3. Media - 900 ml Simulated Gastric Fluid without enzyme (SGF).
4. Analytical Method- Reversed-phase high performance liquid chromatography (HPLC) on a Waters Atlantis dC18 3.0 x 250 mm, 5 µm column maintained at 60°C using a mobile phase consisting of acetonitrile and potassium phosphate monobasic buffer at pH 3.0 with UV detection at 230 nm. Flow rate 1.0 ml/minute.

### Equipment

Waters Alliance 2695 HPLC system with transfer module and 2487 UV-Visible absorbance detector
Waters Empower Software
Hanson SR8 Plus Dissolution Bath

The assay, impurities and dissolution (Method II) results are summarized in Tables 14-2 and 14-3 after one month at 25°C/60% RH and 40°C/75% RH with and without desiccant.

**Table 14-2**

| | | **Assay and Total Impurities Results** | | | | | | | | |
|---|---|---|---|---|---|---|---|---|---|---|
| | **Method** | **Initial** | **1-Month, 25°C/60/% RH** | | **1-Month, 40°C/75% RH** | | **2-Month, 25°C/60% RH** | | **2-Month, 40°C/75% RH** | |
| | | | Without Desiccant | With Desiccant | Without Desiccant | With Desiccant | With-out Desiccant | With Desiccant | With-out Desiccant | With Desiccant |
| **Avg, % Oxycodo ne HCl (n=2)** | Assay | *99.33* | *98.46* | *99.34* | *98.78* | *99.48* | *99.02* | *99.62* | *99.23* | *99.32* |
| **% Total Degradation Products (n=1)** | **Degra-dation Products** | < LOQ | < LOQ | < LOQ | < LOQ | < LOQ | < LOQ | < LOQ | < LOQ | < LOQ |

| | | | | | | | | | | |
|---|---|---|---|---|---|---|---|---|---|---|
| < LOQ= Less Than Limit of Quantitation = 0.1% | | | | | | | | | | |

**Table 14-3**

| | **Dissolution Result (Method II)** | | | | | | |
|---|---|---|---|---|---|---|---|
| | **Mean % Released** | | | | | | |
| **Dissolution Media** | 1 hr. | 2 hrs. | 4 hrs | 8 hrs. | 12 hrs. | 18 hrs. | 24 hrs. |
| **SGF (Initial, n=6)** | 17 | | 42 | 66 | 81 | | |
| **SGF (1-Month 25°C/60% RH without desiccant, n=3)** | 16 | 25 | 40 | 63 | 79 | 92 | 99 |
| **SGF (1-Month 25°C/60% RH with desiccant, n=3)** | 17 | 25 | 41 | 64 | 80 | 93 | 99 |
| **SGF (1-Month 40°C/75% RH without desiccant, n=3)** | 16 | 26 | 42 | 65 | 80 | 93 | 99 |
| **SGF (1-Month 40°C/75% RH with desiccant, n=3)** | 16 | 25 | 41 | 63 | 79 | 92 | 98 |
| **SGF (2-Month 25°C/60% RH without desiccant, n=6)** | 17 | 25 | 41 | 64 | 79 | 92 | 99 |
| **SGF (2-Month 25°C/60% RH with desiccant, n=6)** | 17 | 26 | 41 | 65 | 80 | 94 | 100 |
| **SGF (2-Month 40°C/75% RH without desiccant, n=6)** | 16 | 25 | 40 | 63 | 78 | 91 | 98 |
| **SGF (2-Month 40°C/75% RH with desiccant, n=6)** | 17 | 26 | 42 | 65 | 81 | 94 | 100 |

### Small Volume Extraction Testing

The extraction of oxycodone HCl from 1.5 mm pellets using absolute anhydrous ethanol was evaluated at room temperature.

### Small Volume Extraction Method

1. Extraction Solvent: 30 ml Absolute Anhydrous Ethanol
2. Number of Units: Approximately 2
3. Shaking Time: 1 hour
4. Diluting Solvent: Absolute Anhydrous Ethanol
5. Analysis: UV/Visible Spectrophotometer (wavelength 240 nm)
6. Equipment
   Agilent 8453 UV/Vis Spectrophotometer with ChemStation Software
   Hewlett-Packard Vectra Computer/Windows XP
   Hellma 10 mm Quartz Cell
   Burrell Model 75 Shaker.

An average of 5.6 % oxycodone HCl was extracted.

## Claims

1. A solid oral extended release pharmaceutical dosage form,
comprising a melt formed multi particulate extended release matrix formulation, comprising
at least one poly(ε-caprolactone), and
at least one active agent,
wherein the active agent is an opioid analgesic.

2. The solid oral extended release pharmaceutical dosage form of claim 1, wherein the melt is formed by an extrusion method; or
wherein the melt is formed by a casting method; or
wherein the melt is formed by an injection molding method.

3. The solid oral extended release pharmaceutical dosage form according to claim 1 or 2,
wherein at least one poly(ε-caprolactone) has an approximate number average molecular weight of at least 10,000; or
wherein the at least one poly(ε-caprolactone) has an approximate number average molecular weight of at least 37,000; or
wherein the at least one poly(ε-caprolactone) has an approximate number average molecular weight of between 10,000 to 80,000; or
wherein the at least one poly(ε-caprolactone) has an approximate number average molecular weight of between 37,000 and 80,000.

4. The solid oral extended release pharmaceutical dosage form according to claim 1 or 2, comprising at least a first poly(ε-caprolactone) with an approximate number average molecular weight of between 10,000 and 25,000 and a second poly(ε-caprolactone) with an approximate number average molecular weight of between 37,000 and 80,000.

5. The solid oral extended release pharmaceutical dosage form according to any one of claims 1 to 4, wherein poly(ε-caprolactone) is present at an amount of at least 50 weight-% of the extended release matrix formulation; or
wherein poly(ε-caprolactone) is present at an amount of at least 60 weight-% of the extended release matrix formulation; or
wherein poly(ε-caprolactone)is present at an amount of between 50 and 90 weight-% of the extended release matrix formulation.

6. The solid oral extended release pharmaceutical dosage form according to claim 1 or 2, wherein the at least one poly(ε-caprolactone) has an approximate number average molecular weight of between 37,000 and 80,000 and is present at an amount of between 50 and 90 weight-% of the extended release matrix formulation.

7. The solid oral extended release pharmaceutical dosage form according to any one of claims 1 to 6, wherein the multi particulates have a diameter in the range of 0.1 to 3 mm.

8. The solid oral extended release pharmaceutical dosage form according to any one of claims 1 to 7, wherein the extended release matrix formulation further comprises at least one polyethylene glycol,
wherein the polyethylene glycol is preferably present at an amount of between 1 and 20 weight-%.

9. The solid oral extended release pharmaceutical dosage form according to any one of claims 1 to 8, wherein the extended release matrix formulation further comprises at least one high molecular weight polyethylene oxide,
wherein the high molecular weight polyethylene oxide has a molecular weight of between 1,000,000 and 10,000,000, based on rheological measurements; and/or
wherein the high molecular weight polyethylene oxide is present at an amount of between 5 and 35 weight-%; and/or
wherein the high molecular weight polyethylene oxide is used which has been screened with a screen with a size of 1/10 or less of the average diameter of the resulting melt formed multi particulate extended release formulation; or
with a 100 US mesh screen or a finer screen.

10. The solid oral extended release pharmaceutical dosage form according to any one of claims 1 to 9, wherein the extended release matrix formulation further comprises at least one poloxamer.

11. The solid oral extended release pharmaceutical dosage form according to any one of claims 1 to 10,
wherein the opioid analgesic is preferably selected from the group of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts, hydrates and solvates thereof, and mixtures of any of the foregoing.

12. The solid oral extended release pharmaceutical dosage form of claim 11, wherein the opioid analgesic is selected from the group of codeine, morphine, oxycodone, hydrocodone, hydromorphone, or oxymorphone or pharmaceutically acceptable salts, hydrates and solvates thereof, and mixtures of any of the foregoing.

13. The solid oral extended release pharmaceutical dosage form of claim 12, wherein the opioid analgesic is oxycodone hydrochloride and the dosage form comprises from 5 mg to 500 mg,
preferably 5 mg, 7.5 mg, 10 mg, 15 mg, 20 mg, 30, mg, 40 mg, 45 mg, 50 mg, 60 mg, or 80 mg, 90 mg, 100 mg, 120 mg or 160 mg of oxycodone hydrochloride.

14. The solid oral extended release pharmaceutical dosage form of claim 13, wherein the opioid analgesic is oxycodone hydrochloride having a 14-hydroxycodeinone level of less than 25 ppm, preferably of less than 15 ppm, less than 10 ppm, less than 5 ppm, or less than 1 ppm.

15. The solid oral extended release pharmaceutical dosage form of claim 12, wherein the opioid analgesic is oxymorphone hydrochloride and the dosage form comprises from 1 mg to 500 mg,
preferably 5 mg, 7.5 mg, 10 mg, 15 mg, 20 mg, 30 mg, 40 mg, 45 mg, 50 mg 60 mg, or 80 mg, 90 mg, 100 mg, 120 mg or 160 mg of oxymorphone hydrochloride.

16. The solid oral extended release pharmaceutical dosage form of claim 12, wherein the opioid analgesic is hydromorphone hydrochloride and the dosage form comprises from 1 mg to 100 mg,
preferably 2 mg, 4 mg, 5 mg, 8 mg, 12 mg, 15 mg, 16 mg, 24 mg, 25 mg, 32 mg, 48 mg, 50 mg, 64 mg or 75 mg of hydromorphone hydrochloride.

17. The solid oral extended release pharmaceutical dosage form of any one of claims 1 to 16, which contains active in immediate release form, preferably
wherein the same or different active agents are in extended release and in immediate release forms.

18. The solid oral extended release pharmaceutical dosage form according to any one of claims 1 to 17, wherein the dosage form provides release rates of the active agent in-vitro when measured by the USP Basket Method at 100 rpm at 900 ml simulated gastric fluid at 37 °C, between 12.5 % and 55 % (by wt) active agent released after 1 hour, between 25 % and 65 % (by wt) active agent released after 2 hours, between 45 % and 85 % (by wt) active agent released after 4 hours and between 55 % and 95 % (by wt) active agent released after 6 hours.

19. The solid oral extended release pharmaceutical dosage form of claim 18, wherein the active agent is oxycodone hydrochloride; and/or
wherein the active agent is hydromorphone hydrochloride; and/or
wherein the active agent is oxymorphone hydrochloride.

20. The solid oral extended release pharmaceutical dosage form according to any one of claims 1 to 17, wherein the dosage form provides release rates of the active agent in-vitro when measured by the USP Basket Method at 100 rpm at 900 ml simulated gastric fluid at 37 °C between 10 % and 30% (by wt) active agent released after 2 hour, 40 % and 75 % (by wt) active agent released after 8 hours and no less than 80%(by wt) active agent released after 22 hours,
wherein the active agent is preferably hydromorphone hydrochloride.

21. The solid oral extended release pharmaceutical dosage form according to any one of claims 1 to 20, wherein the dosage form provides an in-vitro dissolution rate, when measured in a USP Apparatus 1 (basket) at 100 rpm in 900 ml simulated gastric fluid comprising 40% ethanol at 37° C, **characterized by** the percent amount of active agent released at 1 hour of dissolution that deviates no more than 20 % points, preferably no more than 10 % points from the corresponding in-vitro dissolution rate measured in a USP Apparatus 1 (basket) at 100 rpm in 900 ml simulated gastric fluid at 37° C without ethanol.

22. The solid oral extended release pharmaceutical dosage form according to any one of claims 1 to 21, wherein the dosage form provides after milling an in-vitro dissolution rate, when measured in a USP Apparatus 1 (basket) at 100 rpm in 900 ml simulated gastric fluid at 37° C, **characterized by** the percent amount of active agent released at 1 hour of dissolution that increases no more than 20 % points, preferably no more than 10 % points when compared to the corresponding in-vitro dissolution rate measured in a USP Apparatus 1 (basket) at 100 rpm in 900 ml simulated gastric fluid at 37° C without milling or decreases when compared to the corresponding in-vitro dissolution rate measured in a USP Apparatus 1 (basket) at 100 rpm in 900 ml simulated gastric fluid at 37° C without milling.

23. The solid oral extended release pharmaceutical dosage form according to any one of claims 1 to 22, wherein the dosage form provides after grinding an in-vitro dissolution rate, when measured in a USP Apparatus 1 (basket) at 100 rpm in 900 ml simulated gastric fluid at 37° C, **characterized by** the percent amount of active agent released at 1 hour of dissolution that increases no more than 20 % points, preferably no more than 10 % points when compared to the corresponding in-vitro dissolution rate measured in a USP Apparatus 1 (basket) at 100 rpm in 900 ml simulated gastric fluid at 37° C without grinding
or decreases when compared to the corresponding in-vitro dissolution rate measured in a USP Apparatus 1 (basket) at 100 rpm in 900 ml simulated gastric fluid at 37° C without grinding.

24. The solid oral extended release pharmaceutical dosage form according to any one of claims 1 to 23, wherein the dosage form after milling provides an in-vitro dissolution rate, when measured in a USP Apparatus 1 (basket) at 100 rpm in 900 ml simulated gastric fluid comprising 40% ethanol at 37° C, **characterized by** the percent amount of active agent released at 1 hour of dissolution that deviates no more than 20 % points, preferably no more than 10 % points from the corresponding in-vitro dissolution rate measured in a USP Apparatus 1 (basket) at 100 rpm in 900 ml simulated gastric fluid without ethanol at 37° C without milling.

25. The solid oral extended release pharmaceutical dosage form according to any one of claims 1 to 24, wherein the dosage form after grinding provides an in-vitro dissolution rate, when measured in a USP Apparatus 1 (basket) at 100 rpm in 900 ml simulated gastric fluid, comprising 40% ethanol, at 37° C, **characterized by** the percent amount of active agent released at 1 hour of dissolution that deviates no more than 20 % points, preferably no more than 10 % points from the corresponding in-vitro dissolution rate measured in a USP Apparatus 1 (basket) at 100 rpm in 900 ml simulated gastric fluid without ethanol at 37° C without grinding.

26. The solid oral extended release pharmaceutical dosage form according to any one of claims 21 to 25, wherein the active agent is oxycodone hydrochloride; and/or
wherein the active agent is hydromorphone hydrochloride; and/or
wherein the active agent is oxymorphone hydrochloride.

27. A solid oral extended release pharmaceutical dosage form including an opioid analgesic and at least one poly(ε-caprolactone) which is resistant to milling and grinding and preferably
wherein the dosage form is resistant to alcohol extraction.

28. A solid extended release pharmaceutical dosage form according to any one of claims 1 to 27 for use in the treatment of pain, wherein the dosage form comprises an opioid analgesic.

29. Use of poly(ε-caprolactone) as matrix forming material in the manufacture of a solid oral extended release pharmaceutical dosage form comprising an active agent selected from opioids for imparting to the solid oral extended release dosage form resistance to milling.

30. A process of preparing a solid oral extended release pharmaceutical dosage form including an active agent selected from the group consisting of opioid analgesics, comprising the steps of:
- Melting and blending the poly(ε-caprolactone) (PCL) and possible further ingredients except the active agent on a Thermodyne Hot Plate (temperature range 90° - 160°C) to obtain a mixture;
- Adding the active agent to the mixture on the Thermodyne Hot Plate (temperature range 90° - 160°C) until the mixture appeared homogeneous to obtain a blend;
- Placing the molten blend on a stainless steel plate and pressing with a second stainless steel plate and cooling to room temperature to obtain a sheet with a given thickness wherein the thickness of the sheet is preferably approximately 2 mm and the pellets have approximately 2 mm in length and width; and
- Pelletizing the sheet by cutting into pellets.

31. A process of preparing a solid oral extended release pharmaceutical dosage form including an active agent selected from the group consisting of opioid analgesics, comprising the steps of:
- Screening active agent, poly(ε-caprolactone) and optionally other ingredients through a # 20 US mesh screen;
- Blending the screened materials at ambient temperature;
- Extruding the screened and blended materials in a twin screw extruder fitted with a die and set on counter-rotation with zone (barrel) temperatures ranged from 18°C to 110°C to obtain strands;
- Cooling the strands to ambient temperature;
- pelletizing the cooled strands into pellets, and
wherein the solid oral extended release pharmaceutical dosage form comprises polyethylene oxide and the polyethylene oxide is screened through a # 100 US mesh screen or finer.

32. A solid oral extended release pharmaceutical dosage form obtainable by a process according to claim 30 or 31.

## Patentansprüche

1. Feste orale verzögert freisetzende (extended release) pharmazeutische Darreichungsform,
umfassend eine schmelzgeformte multipartikuläre verzögert freisetzende Matrixformulierung, umfassend
mindestens ein Poly(ε-caprolacton), und
mindestens einen Wirkstoff,
wobei der Wirkstoff ein Opioidanalgetikum ist.

2. Die feste orale verzögert freisetzende pharmazeutische Darreichungsform gemäß Anspruch 1, wobei die Schmelze durch ein Extrusionsverfahren geformt ist; oder
wobei die Schmelze durch ein Gießverfahren geformt ist; oder
wobei die Schmelze durch Spritzgussverfahren geformt ist.

3. Die feste orale verzögert freisetzende pharmazeutische Darreichungsform gemäß Anspruch 1 oder 2, wobei mindestens ein Poly(ε-caprolacton) ein ungefähres durchschnittliches Molekulargewicht (Zahlenmittel) von mindestens 10.000 hat; oder
wobei das mindestens eine Poly(ε-caprolacton) ein ungefähres durchschnittliches Molekulargewicht (Zahlenmittel) von mindestens 37.000 hat; oder
wobei das mindestens eine Poly(ε-caprolacton) ein ungefähres durchschnittliches Molekulargewicht (Zahlenmittel) von zwischen 10.000 und 80.000 hat; oder
wobei das mindestens eine Poly(ε-caprolacton) ein ungefähres durchschnittliches Molekulargewicht (Zahlenmittel) von zwischen 37.000 und 80.000 hat.

4. Die feste orale verzögert freisetzende pharmazeutische Darreichungsform gemäß Anspruch 1 oder 2, umfassend mindestens ein erstes Poly(ε-caprolacton) mit einem ungefähren durchschnittlichen Molekulargewicht (Zahlenmittel) von zwischen 10.000 und 25.000 und ein zweites Poly(ε-caprolacton) mit einem ungefähren durchschnittlichen Molekulargewicht (Zahlenmittel) von zwischen 37.000 und 80.000.

5. Die feste orale verzögert freisetzende pharmazeutische Darreichungsform gemäß einem der Ansprüche 1 bis 4, wobei Poly(ε-caprolacton) in einer Menge von mindestens 50 Gewichts-% der verzögert freisetzenden Matrixformulierung vorhanden ist; oder
wobei Poly(ε-caprolacton) in einer Menge von mindestens 60 Gewichts-% der verzögert freisetzenden Matrixformulierung vorhanden ist; oder
wobei Poly(ε-caprolacton) in einer Menge von zwischen 50 und 90 Gewichts-% der verzögert freisetzenden Matrixformulierung vorhanden ist.

6. Die feste orale verzögert freisetzende pharmazeutische Darreichungsform gemäß Anspruch 1 oder 2, wobei das mindestens eine Poly(ε-caprolacton) ein ungefähres durchschnittliches Molekulargewicht (Zahlenmittel) von zwischen 37.000 und 80.000 hat und in einer Menge von zwischen 50 und 90 Gewichts-% der verzögert freisetzenden Matrixformulierung vorhanden ist.

7. Die feste orale verzögert freisetzende pharmazeutische Darreichungsform gemäß einem der Ansprüche 1 bis 6, wobei die Multipartikel einen Durchmesser im Bereich von 0,1 bis 3 mm haben.

8. Die feste orale verzögert freisetzende pharmazeutische Darreichungsform gemäß einem der Ansprüche 1 bis 7, wobei die verzögert freisetzende Matrixformulierung ferner mindestens ein Polyethylenglycol umfasst,
wobei das Polyethylenglycol vorzugsweise in einer Menge von zwischen 1 und 20 Gewichts-% vorhanden ist.

9. Die feste orale verzögert freisetzende pharmazeutische Darreichungsform gemäß einem der Ansprüche 1 bis 8, wobei die verzögert freisetzende Matrixformulierung ferner mindestens ein hochmolekulares Polyethylenoxid umfasst,
wobei das hochmolekulare Polyethylenoxid ein Molekulargewicht basierend auf rheologischen Messungen von zwischen 1.000.000 und 10.000.000 hat; und/oder
wobei das hochmolekulare Polyethylenoxid in einer Menge zwischen 5 und 35 Gewichts-% vorhanden ist; und/oder
wobei das hochmolekulare Polyethylenoxid benutzt wird, das mit einem Sieb mit einer Abmessung von 1/10 oder weniger des durchschnittlichen Durchmessers der entstehenden schmelzgeformten, multipartikulären, verzögert freisetzenden Formulierung gesiebt wurde; oder
mit einem Sieb mit 100 US Mesh oder einem feinmaschigerem Sieb.

10. Die feste orale verzögert freisetzende pharmazeutische Darreichungsform gemäß einem der Ansprüche 1 bis 9, wobei die verzögert freisetzende Matrixformulierung ferner mindestens ein Poloxamer umfasst.

11. Die feste orale verzögert freisetzende pharmazeutische Darreichungsform gemäß einem der Ansprüche 1 bis 10,
wobei das Opioidanalgetikum vorzugsweise ausgewählt ist aus der Gruppe von Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etorphin, Dihydroetorphin, Fentanyl und Derivate, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normophin, Norpipanon, Opium, Oxycodon, Oxymorphon, Papaveretum, Pentazocin, Phenadoxon, Phenomorphan, Phenazocin, Phenoperidin, Piminodin, Piritramid, Propheptazin, Promedol, Properidin, Propoxyphen, Sufentanil, Tilidin, Tramadol, pharmazeutisch akzeptable Salze, Hydrate und Solvate davon, sowie sämtliche Mischungen.

12. Die feste orale verzögert freisetzende pharmazeutische Darreichungsform gemäß Anspruch 11, wobei das Opioidanalgetikum ausgewählt ist aus der Gruppe von Codein, Morphin, Oxycodon, Hydrocodon, Hydromorphon oder Oxymorphon oder pharmazeutisch akzeptable Salze, Hydrate und Solvate davon, sowie sämtliche Mischungen.

13. Die feste orale verzögert freisetzende pharmazeutische Darreichungsform gemäß Anspruch 12, wobei das Opioidanalgetikum Oxycodonhydrochlorid ist und die Darreichungsform von 5 mg bis 500 mg,
vorzugsweise 5 mg, 7,5 mg, 10 mg, 15 mg, 20 mg, 30, mg, 40 mg, 45 mg, 50 mg, 60 mg oder 80 mg, 90 mg, 100 mg, 120 mg oder 160 mg Oxycodonhydrochlorid umfasst.

14. Die feste orale verzögert freisetzende pharmazeutische Darreichungsform gemäß Anspruch 13, wobei das Opioidanalgetikum Oxycodonhydrochlorid ist, das einen 14-Hydroxycodeinon-Gehalt von weniger als 25 ppm, vorzugsweise von weniger als 15 ppm, weniger als 10 ppm, weniger als 5 ppm, oder weniger als 1 ppm aufweist.

15. Die feste orale verzögert freisetzende pharmazeutische Darreichungsform gemäß Anspruch 12, wobei das Opioidanalgetikum Oxymorphonhydrochlorid ist und die Darreichungsform von 1 mg bis 500 mg,
vorzugsweise 5 mg, 7,5 mg, 10 mg, 15 mg, 20 mg, 30 mg, 40 mg, 45 mg, 50 mg, 60 mg, oder 80 mg, 90 mg, 100 mg, 120 mg oder 160 mg Oxymorphonhydrochlorid umfasst.

16. Die feste orale verzögert freisetzende pharmazeutische Darreichungsform gemäß Anspruch 12, wobei das Opioidanalgetikum Hydromorphonhydrochlorid ist und die Darreichungsform von 1 mg bis 100 mg,
vorzugsweise 2 mg, 4 mg, 5 mg, 8 mg, 12 mg, 15 mg, 16 mg, 24 mg, 25 mg, 32 mg, 48 mg, 50 mg, 64 mg oder 75 mg Hydromorphonhydrochlorid umfasst.

17. Die feste orale verzögert freisetzende pharmazeutische Darreichungsform gemäß einem der Ansprüche 1 bis 16, die Wirkstoff in sofort freisetzender Form umfasst, vorzugsweise
wobei die gleichen oder verschiedene Wirkstoffe in verzögert freisetzender und sofort freisetzender Form sind.

18. Die feste orale verzögert freisetzende pharmazeutische Darreichungsform gemäß einem der Ansprüche 1 bis 17, wobei die Darreichungsform eine in-vitro Freisetzungsgeschwindigkeit des Wirkstoffs, wenn sie mittels USP Körbchen-Verfahren bei 100 Upm in 900 ml simulierter Magenflüssigkeit bei 37°C gemessen wird, zwischen 12,5 % und 55 Gew.-% freigesetzten Wirkstoffs nach 1 Std., zwischen 25 % und 65 Gew.-% freigesetzten Wirkstoffs nach 2 Std., zwischen 45 und 85 Gew.-% freigesetzten Wirkstoffs nach 4 Std. und zwischen 55 und 95 Gew.-% freigesetzten Wirkstoffs nach 6 Std. bereitstellt.

19. Die feste orale verzögert freisetzende pharmazeutische Darreichungsform gemäß Anspruch 18, wobei der Wirkstoff Oxycodonhydrochlorid ist; und/oder
wobei der Wirkstoff Hydromorphonhydrochlorid ist; und/oder
wobei der Wirkstoff Oxymorphonhydrochlorid ist.

20. Die feste orale verzögert freisetzende pharmazeutische Darreichungsform gemäß einem der Ansprüche 1 bis 17, wobei die Darreichungsform eine in-vitro Freisetzungsgeschwindigkeit des Wirkstoffs, wenn sie mittels USP Körbchen-Verfahren bei 100 Upm in 900 ml simulierter Magenflüssigkeit bei 37°C gemessen wird, zwischen 10 % und 30 Gew.-% freigesetzten Wirkstoffs nach 2 Std., 40 und 75 Gew.-% freigesetzten Wirkstoffs nach 8 Std. und nicht weniger als 80 Gew.-% freigesetzten Wirkstoffs nach 22 Std. bereitstellt,
wobei der Wirkstoff vorzugsweise Hydromorphonhydrochlorid ist.

21. Die feste orale verzögert freisetzende pharmazeutische Darreichungsform gemäß einem der Ansprüche 1 bis 20, wobei die Darreichungsform eine in-vitro Auflösungsgeschwindigkeit bereitstellt, die, wenn sie in einem USP-Apparat 1 (Körbchen) bei 100 Upm in 900 ml simulierter Magenflüssigkeit umfassend 40 % Ethanol bei 37°C gemessen wird, dadurch charakterisiert ist, dass die prozentuale Menge an freigesetztem Wirkstoff bei 1 Std. Auflösung nicht mehr als 20 %-Punkte, vorzugsweise nicht mehr als 10 %-Punkte von der entsprechenden in-vitro Auflösungsgeschwindigkeit abweicht, die in einem USP-Apparat 1 (Körbchen) bei 100 Upm in 900 ml simulierter Magenflüssigkeit bei 37°C ohne Ethanol gemessen wird.

22. Die feste orale verzögert freisetzende pharmazeutische Darreichungsform gemäß einem der Ansprüche 1 bis 21, wobei die Darreichungsform nach Mahlen eine in-vitro Auflösungsgeschwindigkeit bereitstellt, die, wenn sie in einem USP-Apparat 1 (Körbchen) bei 100 Upm in 900 ml simulierter Magenflüssigkeit bei 37°C gemessen wird, dadurch charakterisiert ist, dass die prozentuale Menge an freigesetztem Wirkstoff bei 1 Std. Auflösung nicht mehr als 20 %-Punkte, vorzugsweise nicht mehr als 10 %-Punkte zunimmt im Vergleich zu der entsprechenden in-vitro Auflösungsgeschwindigkeit, die in einem USP-Apparat 1 (Körbchen) bei 100 Upm in 900 ml simulierter Magenflüssigkeit bei 37°C ohne Mahlen gemessen wird, oder abnimmt im Vergleich zu der entsprechenden in-vitro Auflösungsgeschwindigkeit, die in einem USP-Apparat 1 (Körbchen) bei 100 Upm in 900 ml simulierter Magenflüssigkeit bei 37°C ohne Mahlen gemessen wird.

23. Die feste orale verzögert freisetzende pharmazeutische Darreichungsform gemäß einem der Ansprüche 1 bis 22, wobei die Darreichungsform nach Zerkleinern eine in-vitro Auflösungsgeschwindigkeit bereitstellt, die, wenn sie in einem USP-Apparat 1 (Körbchen) bei 100 Upm in 900 ml simulierter Magenflüssigkeit bei 37°C gemessen wird, dadurch charakterisiert ist, dass die prozentuale Menge an freigesetztem Wirkstoff bei 1 Std. Auflösung nicht mehr als 20 %-Punkte, vorzugsweise nicht mehr als 10 %-Punkte zunimmt im Vergleich zu der entsprechenden in-vitro Auflösungsgeschwindigkeit, die in einem USP-Apparat 1 (Körbchen) bei 100 Upm in 900 ml simulierter Magenflüssigkeit bei 37°C ohne Zerkleinern gemessen wird,
oder abnimmt im Vergleich zu der entsprechenden in-vitro Auflösungsgeschwindigkeit, die in einem USP-Apparat 1 (Körbchen) bei 100 Upm in 900 ml simulierter Magenflüssigkeit bei 37°C ohne Zerkleinern gemessen wird.

24. Die feste orale verzögert freisetzende pharmazeutische Darreichungsform gemäß einem der Ansprüche 1 bis 23, wobei die Darreichungsform nach Mahlen eine in-vitro Auflösungsgeschwindigkeit bereitstellt, die, wenn sie in einem USP-Apparat 1 (Körbchen) bei 100 Upm in 900 ml simulierter Magenflüssigkeit umfassend 40 % Ethanol bei 37°C gemessen wird, dadurch charakterisiert ist, dass die prozentuale Menge an freigesetztem Wirkstoff bei 1 Std. Auflösung nicht mehr als 20 %-Punkte, vorzugsweise nicht mehr als 10 %-Punkte von der entsprechenden in-vitro Auflösungsgeschwindigkeit abweicht, die in einem USP-Apparat 1 (Körbchen) bei 100 Upm in 900 ml simulierter Magenflüssigkeit ohne Ethanol bei 37°C ohne Mahlen gemessen wird.

25. Die feste orale verzögert freisetzende pharmazeutische Darreichungsform gemäß einem der Ansprüche 1 bis 24, wobei die Darreichungsform nach Zerkleinern eine in-vitro Auflösungsgeschwindigkeit bereitstellt, die, wenn sie in einem USP-Apparat 1 (Körbchen) bei 100 Upm in 900 ml simulierter Magenflüssigkeit umfassend 40 % Ethanol bei 37°C gemessen wird, dadurch charakterisiert ist, dass die prozentuale Menge an freigesetztem Wirkstoff bei 1 Std. Auflösung nicht mehr als 20 %-Punkte, vorzugsweise nicht mehr als 10 %-Punkte von der entsprechenden in-vitro Auflösungsgeschwindigkeit abweicht, die in einem USP-Apparat 1 (Körbchen) bei 100 Upm in 900 ml simulierter Magenflüssigkeit ohne Ethanol bei 37°C ohne Zerkleinern gemessen wird.

26. Die feste orale verzögert freisetzende pharmazeutische Darreichungsform gemäß einem der Ansprüche 21 bis 25, wobei der Wirkstoff Oxycodonhydrochlorid ist; und/oder
wobei der Wirkstoff Hydromorphonhydrochlorid ist; und/oder wobei der Wirkstoff Oxymorphonhydrochlorid ist.

27. Feste orale verzögert freisetzende pharmazeutische Darreichungsform enthaltend ein Opioidanalgetikum und mindestens ein Poly(ε-caprolacton), welche widerstandsfähig gegen Mahlen und Zerkleinern ist und vorzugsweise
wobei die Darreichungsform widerstandsfähig gegen Alkoholextraktion ist.

28. Die feste verzögert freisetzende pharmazeutische Darreichungsform gemäß einem der Ansprüche 1 bis 27 zur Verwendung für die Behandlung von Schmerz, wobei die Darreichungsform ein Opioidanalgetikum umfasst.

29. Verwendung von Poly(ε-caprolacton) als Matrix-bildendes Material in der Herstellung einer festen oralen verzögert freisetzenden pharmazeutischen Darreichungsform umfassend einen Wirkstoff ausgewählt aus Opioiden zur Verleihung von Resistenz gegen Mahlen an die feste verzögert freisetzende pharmazeutische Darreichungsform.

30. Verfahren zur Herstellung einer festen oralen verzögert freisetzenden pharmazeutischen Darreichungsform enthaltend einen Wirkstoff ausgewählt aus der Gruppe bestehend aus Opioidanalgetika, umfassend die Schritte aus:
- Schmelzen und vermengen des Poly(ε-caprolacton) (PCL) und möglicher weiterer Bestandteile ausgenommen des Wirkstoffs auf einer Thermodyne Heizplatte (Temperaturbereich 90° - 160°C), um eine Mischung zu erhalten;
- Zugeben des Wirkstoffs zu der Mischung auf der Thermodyne Heizplatte (Temperaturbereich 90° - 160°C) bis die Mischung homogen erscheint, um ein Gemisch zu erhalten;
- Platzieren des geschmolzenen Gemischs auf einer Edelstahlplatte und aufpressen mit einer zweiten Edelstahlplatte und abkühlen auf Raumtemperatur um eine Schicht mit bestimmter Dicke zu erhalten, wobei die Dicke der Schicht vorzugsweise ungefähr 2 mm beträgt und das Granulat eine Länge und Breite von ungefähr 2 mm hat; und
- Granulieren der Schicht durch Zerschneiden in Granulate.

31. Verfahren zur Herstellung einer festen oralen verzögert freisetzenden pharmazeutischen Darreichungsform enthaltend einen Wirkstoff ausgewählt aus der Gruppe bestehend aus Opioidanalgetika, umfassend die Schritte aus:
- Sieben des Wirkstoffs, des Poly(ε-caprolacton) und optional anderer Bestandteile durch ein Sieb mit 20 US Mesh;
- Vermengen der gesiebten Materialien bei Raumtemperatur;
- Extrudieren der gesiebten und vermengten Materialien in einem Doppelschneckenextruder ausgestattet mit einer Düse und betrieben auf Gegenlauf mit Zonen (Zylinder)-Temperaturen von 18°C bis 110°C, um Stränge zu erhalten;
- Abkühlen der Stränge auf Raumtemperatur;
- Granulieren der abgekühlten Stränge in Granulate, und
wobei die feste orale verzögert freisetzende pharmazeutische Darreichungsform ein Polyethylenoxid umfasst und das Polyethylenoxid durch ein Sieb mit 100 US Mesh oder feiner gesiebt ist.

32. Feste orale verzögert freisetzende pharmazeutische Darreichungsform, erhältlich durch ein Verfahren gemäß Anspruch 30 oder 31.

## Revendications

1. Forme posologique pharmaceutique orale solide à libération prolongée, comprenant une formulation de matrice à libération prolongée formée à partir d'une masse fondue, à particules multiples, comprenant
au moins une poly(ε-caprolactone) et
au moins un agent actif,
dans laquelle l'agent actif est un analgésique opioïde.

2. Forme posologique pharmaceutique orale solide à libération prolongée suivant la revendication 1, dans laquelle la masse fondue est formée par un procédé d'extrusion ; ou
dans laquelle la masse fondue est formée par un procédé de coulée ; ou
dans laquelle la masse fondue est formée par un procédé de moulage par injection.

3. Forme posologique pharmaceutique orale solide à libération prolongée suivant la revendication 1 ou 2, dans laquelle au moins une poly(ε-caprolactone) a une moyenne en nombre du poids moléculaire approximative d'au moins 10 000 ; ou
dans laquelle ladite au moins une poly(ε-caprolactone) a une moyenne en nombre du poids moléculaire approximative d'au moins 37 000 ; ou
dans laquelle ladite au moins une poly(ε-caprolactone) a une moyenne en nombre du poids moléculaire approximative entre 10 000 et 80 000 ; ou
dans laquelle ladite au moins une poly(ε-caprolactone) a une moyenne en nombre du poids moléculaire approximative entre 37 000 et 80 000.

4. Forme posologique pharmaceutique orale solide à libération prolongée suivant la revendication 1 ou 2, comprenant au moins une première poly(ε-caprolactone) ayant une moyenne en nombre du poids moléculaire approximative entre 10 000 et 25 000 et une seconde poly(ε-caprolactone) ayant une moyenne en nombre du poids moléculaire approximative entre 37 000 et 80 000.

5. Forme posologique pharmaceutique orale solide à libération prolongée suivant l'une quelconque des revendications 1 à 4, dans laquelle la ou les poly(ε-caprolactone)(s) est/sont présente(s) en une quantité d'au moins 50 % en poids de la formulation de matrice à libération prolongée ; ou
dans laquelle la ou les poly(ε-caprolactone)(s) est/sont présente(s) en une quantité d'au moins 60 % en poids de la formulation de matrice à libération prolongée ; ou
dans laquelle la ou les poly(ε-caprolactone)(s) est/sont présente(s) en une quantité entre 50 et 90 % en poids de la formulation de matrice à libération prolongée.

6. Forme posologique pharmaceutique orale solide à libération prolongée suivant la revendication 1 ou 2, dans laquelle ladite au moins une poly(ε-caprolactone) a une moyenne en nombre du poids moléculaire approximative entre 37 000 et 80 000 et est présente en une quantité entre 50 et 90 % en poids de la formulation de matrice à libération prolongée.

7. Forme posologique pharmaceutique orale solide à libération prolongée suivant l'une quelconque des revendications 1 à 6, dans laquelle les particules multiples ont un diamètre dans l'intervalle de 0,1 à 3 mm.

8. Forme posologique pharmaceutique orale solide à libération prolongée suivant l'une quelconque des revendications 1 à 7, dans laquelle la formulation de matrice à libération prolongée comprend en outre au moins un polyéthylèneglycol,
le polyéthylèneglycol étant de préférence présent en une quantité entre 1 et 20 % en poids.

9. Forme posologique pharmaceutique orale solide à libération prolongée suivant l'une quelconque des revendications 1 à 8, dans laquelle la formulation de matrice à libération prolongée comprend en outre au moins un poly(oxyde d'éthylène) de haut poids moléculaire,
dans laquelle le poly(oxyde d'éthylène) de haut poids moléculaire a un poids moléculaire entre 1 000 000 et 10 000 000, sur la base de mesures rhéologiques ; et/ou
dans laquelle le poly(oxyde d'éthylène) de haut poids moléculaire est présent en une quantité entre 5 et 35 % en poids ; et/ou
dans laquelle le poly(oxyde d'éthylène) de haut poids moléculaire est utilisé qui a été tamisé avec un tamis ayant des mailles égales ou inférieures à 1/10 du diamètre moyen de la formulation résultante à libération prolongée en particules multiples formée à partir d'une masse fondue; ou
avec un tamis à mailles US n° 100 ou un tamis plus fin.

10. Forme posologique pharmaceutique orale solide à libération prolongée suivant l'une quelconque des revendications 1 à 9, dans laquelle la formulation de matrice à libération prolongée comprend en outre au moins un poloxamère.

11. Forme posologique pharmaceutique orale solide à libération prolongée suivant l'une quelconque des revendications 1 à 10,
dans laquelle l'analgésique opioïde est choisi de préférence dans le groupe comprenant l'alfentanil, l'allylprodine, l'alphaprodine, l'aniléridine, la benzylmorphine, le bézitramide, la buprénorphine, le butorphanol, le clonitazène, la codéine, la désomorphine, le dextromoramide, la dézocine, le diampromide, la diamorphone, la dihydrocodéine, la dihydromorphine, le diménoxadol, le dimépheptanol, le diméthyl-thiambutène, le butyrate de dioxaphétyl, la dipinanone, l'eptazocine, l'éthoheptazine, l'éthylméthylthiambutène, l'éthyl-morphine, l'étonitazène, l'étorphine, la dihydroétorphine, le fentanyl et ses dérivés, l'hydrocodone, l'hydromorphone, l'hydroxypéthidine, l'isométhadone, la kétobémidone, le lévorphanol, le lévophénacylmorphane, le lofentanil, la mépéridine, le meptazinol, la métazocine, la méthadone, le métopon, la morphine, la myrophine, la narcéine, la nicomorphine, le norlévorphanol, la norméthadone, la nalorphine, le nalbuphéne, la normorphine, la norpipanone, l'opium, l'oxycodone, l'oxymorphone, le papaveretum, la pentazocine, la phénadoxone, le phénomorphane, la phénazocine, la phénopéridine, la piminodine, le piritramide, la propheptazine, le promédol, la propéridine, le propoxyphène, le sufentanil, la tilidine, le tramadol, leurs sels, hydrates et produits de solvatation pharmaceutiquement acceptables, ainsi que des mélanges de n'importe lesquels des agents précités.

12. Forme posologique pharmaceutique orale solide à libération prolongée suivant la revendication 11, dans laquelle l'analgésique opioïde est choisi dans le groupe comprenant la codéine, la morphine, l'oxycodone, l'hydrocodone, l'hydromorphone, l'oxymorphone ou leurs sels, hydrates et produits de solvatation pharmaceutiquement acceptables, ainsi que des mélanges de n'importe lesquels des agents précités.

13. Forme posologique pharmaceutique orale solide à libération prolongée suivant la revendication 12, dans laquelle l'analgésique opioïde est le chlorhydrate d'oxycodone et la forme posologique comprend 5 mg à 500 mg,
de préférence 5 mg, 7,5 mg, 10 mg, 15 mg, 20 mg, 30 mg, 40 mg, 45 mg, 50 mg, 60 mg ou 80 mg, 90 mg, 100 mg, 120 mg ou 160 mg de chlorhydrate d'oxycodone.

14. Forme posologique pharmaceutique orale solide à libération prolongée suivant la revendication 13, dans laquelle l'analgésique opioïde est le chlorhydrate d'oxycodone ayant une teneur en 14-hydroxycodéinone inférieure à 25 ppm, de préférence inférieure à 15 ppm, inférieure à 10 ppm, inférieure à 5 ppm ou inférieure à 1 ppm.

15. Forme posologique pharmaceutique orale solide à libération prolongée suivant la revendication 12, dans laquelle l'analgésique opioïde est le chlorhydrate d'oxymorphone et la forme posologique comprend 1 mg à 500 mg,
de préférence 5 mg, 7,5 mg, 10 mg, 15 mg, 20 mg, 30 mg, 40 mg, 45 mg, 50 mg, 60 mg ou 80 mg, 90 mg, 100 mg, 120 mg ou 160 mg de chlorhydrate d'oxymorphone.

16. Forme posologique pharmaceutique orale solide à libération prolongée suivant la revendication 12, dans laquelle l'analgésique opioïde est le chlorhydrate d'hydromorphone et la forme posologique comprend 1 mg à 100 mg,
de préférence 2 mg, 4 mg, 5 mg, 8 mg, 12 mg, 15 mg, 16 mg, 24 mg, 25 mg, 32 mg, 48 mg, 50 mg, 64 mg ou 75 mg de chlorhydrate d'hydromorphone.

17. Forme posologique pharmaceutique orale solide à libération prolongée suivant l'une quelconque des revendications 1 à 16, qui contient un agent actif dans la forme à libération immédiate, de préférence
dans laquelle le même agent actif ou des agents actifs différents sont présents dans la forme à libération prolongée et la forme à libération immédiate.

18. Forme posologique pharmaceutique orale solide à libération prolongée suivant l'une quelconque des revendications 1 à 17, ladite forme posologique présentant des vitesses de libération de l'agent actif in vitro, lors de la mesure par le procédé à panier USP à 100 tr/min dans 900 ml de fluide gastrique simulé à 37°C, entre 12,5 % et 55 % (en poids) d'agent actif libérés au bout de 1 heure, entre 25 % et 65 % (en poids) d'agent actif libéré au bout de 2 heures, entre 45 % et 85 % (en poids) d'agent actif libérés au bout de 4 heures et entre 55 % et 95 % (en poids) d'agent actif libérés au bout de 6 heures.

19. Forme posologique pharmaceutique orale solide à libération prolongée suivant la revendication 18, dans laquelle l'agent actif est le chlorhydrate d'oxycodone ; et/ou
dans laquelle l'agent actif est le chlorhydrate d'hydromorphone ; et/ou
dans laquelle l'agent actif est le chlorhydrate d'oxymorphone.

20. Forme posologique pharmaceutique orale solide à libération prolongée suivant l'une quelconque des revendications 1 à 17, ladite forme posologique présentant des vitesses de libération de l'agent actif in vitro, lors de la mesure par le procédé à panier USP à 100 tr/min dans 900 ml de fluide gastrique simulé à 37°C, entre 10 % et 30 % (en poids) d'agent actif libérés au bout de 2 heures, 40 % et 75 % (en poids) d'agent actif libérés au bout de 8 heures, et pas moins de 80 % (en poids) d'agent actif libérés au bout de 22 heures,
l'agent actif étant de préférence le chlorhydrate d'hydromorphone.

21. Forme posologique pharmaceutique orale solide à libération prolongée suivant l'une quelconque des revendications 1 à 20, ladite forme posologique présentant une vitesse de dissolution in vitro, lors de la mesure dans un appareil USP 1 (à panier) à 100 tr/min dans 900 ml de fluide gastrique simulé comprenant 40 % d'éthanol à 37°C, **caractérisée par** la quantité en pourcentage d'agent actif libérée au bout de 1 heure de dissolution qui s'écarte de pas plus de 20 points de pourcent, de préférence de pas plus de 10 points de pourcent, de la vitesse de dissolution in vitro correspondante mesurée dans un appareil USP 1 (à panier) à 100 tr/min dans 900 ml de fluide gastrique simulé à 37°C, sans éthanol.

22. Forme posologique pharmaceutique orale solide à libération prolongée suivant l'une quelconque des revendications 1 à 21, ladite forme posologique présentant, après broyage, une vitesse de dissolution in vitro, lors de la mesure dans un appareil USP 1 (à panier) à 100 tr/min dans 900 ml de fluide gastrique simulé à 37°C, **caractérisée par** la quantité en pourcentage d'agent actif libérée au bout de 1 heure de dissolution qui augmente de pas plus de 20 points de pourcent, de préférence de pas plus de 10 points de pourcent, comparativement à la vitesse de dissolution in vitro correspondante mesurée dans un appareil USP 1 (à panier) à 100 tr/min dans 900 ml de fluide gastrique simulé à 37°C, sans broyage ou qui diminue comparativement à la vitesse de dissolution in vitro correspondante mesurée dans un appareil USP 1 (à panier) à 100 tr/min dans 900 ml de fluide gastrique simulé à 37°C, sans broyage.

23. Forme posologique pharmaceutique orale solide à libération prolongée suivant l'une quelconque des revendications 1 à 22, ladite forme posologique présentant, après mouture, une vitesse de dissolution in vitro, lors de la mesure dans un appareil USP 1 (à panier) à 100 tr/min dans 900 ml de fluide gastrique simulé à 37°C, **caractérisée par** la quantité en pourcentage d'agent actif libérée au bout de 1 heure de dissolution qui augmente de pas plus de 20 points de pourcent, de préférence de pas plus de 10 points de pourcent, comparativement à la vitesse de dissolution in vitro correspondante mesurée dans un appareil USP 1 (à panier) à 100 tr/min dans 900 ml de fluide gastrique simulé à 37°C, sans mouture,
ou qui diminue comparativement à la vitesse de dissolution in vitro correspondante mesurée dans un appareil USP 1 (à panier) à 100 tr/min dans 900 ml de fluide gastrique simulé à 37°C, sans mouture.

24. Forme posologique pharmaceutique orale solide à libération prolongée suivant l'une quelconque des revendications 1 à 23, ladite forme posologique, après broyage, présentant une vitesse de dissolution in vitro, lors de la mesure dans un appareil USP 1 (à panier) à 100 tr/min dans 900 ml de fluide gastrique simulé comprenant 40 % d'éthanol, à 37°C, **caractérisée par** la quantité en pourcentage d'agent actif libérée au bout de 1 heure de dissolution qui s'écarte de pas plus de 20 points de pourcent, de préférence de pas plus de 10 points de pourcent, de la vitesse de dissolution in vitro correspondante mesurée dans un appareil USP 1 (à panier) à 100 tr/min dans 900 ml de fluide gastrique simulé sans éthanol à 37°C, sans broyage.

25. Forme posologique pharmaceutique orale solide à libération prolongée suivant l'une quelconque des revendications 1 à 24, ladite forme posologique, après mouture, présentant une vitesse de dissolution in vitro, lors de la mesure dans un appareil USP 1 (à panier) à 100 tr/min dans 900 ml de fluide gastrique simulé comprenant 40 % d'éthanol, à 37°C, **caractérisée par** la quantité en pourcentage d'agent actif libérée au bout de 1 heure de dissolution qui s'écarte de pas plus de 20 points de pourcent, de préférence de pas plus de 10 points de pourcent, de la vitesse de dissolution in vitro correspondante mesurée dans un appareil USP 1 (à panier) à 100 tr/min dans 900 ml de fluide gastrique simulé sans éthanol à 37°C, sans mouture.

26. Forme posologique pharmaceutique orale solide à libération prolongée suivant l'une quelconque des revendications 21 à 25, dans laquelle l'agent actif est le chlorhydrate d'oxycodone ; et/ou
dans laquelle l'agent actif est le chlorhydrate d'hydromorphone ; et
dans laquelle l'agent actif est le chlorhydrate d'oxymorphone.

27. Forme posologique pharmaceutique orale solide à libération prolongée comprenant un analgésique opioïde et au moins une poly(ε-caprolactone), qui est résistante au broyage et à la mouture,
ladite forme posologique étant de préférence résistante à l'extraction par l'alcool.

28. Forme posologique pharmaceutique solide à libération prolongée suivant l'une quelconque des revendications 1 à 27, pour une utilisation dans le traitement de la douleur, ladite forme posologique comprenant un analgésique opioïde.

29. Utilisation de poly(ε-caprolactone) comme matière de formation de matrice dans la production d'une forme posologique pharmaceutique orale solide à libération prolongée comprenant un agent actif choisi parmi des opioïdes pour conférer à la forme posologique pharmaceutique orale solide à libération prolongée une résistance au broyage.

30. Procédé de préparation d'une forme posologique pharmaceutique orale solide à libération prolongée incluant un agent actif choisi dans le groupe consistant en analgésiques opioïdes, comprenant les étapes de :
- fusion et mélange de la poly(ε-caprolactone) (PCL) et d'ingrédients supplémentaires éventuels à l'exception de l'agent actif sur une plaque chauffante Thermodyne (plage de températures 90°-160°C) pour obtenir un mélange ;
- addition de l'agent actif au mélange sur la plaque chauffante Thermodyne (plage de températures 90°-160°C) jusqu'à ce que le mélange ait un aspect homogène pour obtenir une formulation ;
- mise en place de la formulation fondue sur une plaque en acier inoxydable et pression avec une seconde plaque en acier inoxydable et refroidissement à température ambiante pour obtenir une feuille ayant une épaisseur donnée, l'épaisseur de la feuille étant de préférence approximativement 2 mm et les granules ayant approximativement 2 mm de longueur et de largeur ; et
- granulation de la feuille par coupe en granules.

31. Procédé de préparation d'une forme posologique pharmaceutique orale solide à libération prolongée comprenant un agent actif choisi dans le groupe consistant en analgésiques opioïdes, comprenant les étapes de :
- tamisage de l'agent actif, de la poly(ε-caprolactone) et éventuellement d'autres ingrédients à travers un tamis à mailles US n° 20 ;
- mélange des matières tamisées à température ambiante ;
- extrusion des matières tamisées et mélangées dans une extrudeuse à deux vis équipée d'une filière et réglée en contre-rotation avec des températures de zone (cylindre) allant de 18°C à 110°c pour obtenir des brins ;
- refroidissement des brins à température ambiante ;
- granulation des brins refroidis en granules, et
dans lequel la forme posologique pharmaceutique orale solide à libération prolongée comprend du poly(oxyde d'éthylène) et le poly(oxyde d'éthylène) est tamisé à travers un tamis à mailles US n° 100 ou plus fin.

32. Forme posologique pharmaceutique orale solide à libération prolongée pouvant être obtenue par un procédé suivant la revendication 30 ou 31.
